# EUROPEAN PATENT APPLICATION

(11) **EP 2 767 273 A1**
(43) Date of publication of application: **20.08.2014**
(21) Application number: 13155510.4
(22) Date of filing: 15.02.2013
(51) Int. Cl.: A61K 31/00, A61K 31/4406, A61P 35/00, A61P 29/00

(54) **Death-associated protein kinases, inhibitors and activators thereof for use in pharmaceutical compositions and in predictive medicine**

(71) Applicant: UNIVERSITÄTSKLINIKUM ERLANGEN, 91054 Erlangen (DE)
(72) Inventor: Schneider-Stock, Regine, 91086 Aurachtal (DE); Neufert, Clemens, 91052 Erlangen (DE)
(74) Representative: Isarpatent

(57) **Abstract**

The present invention concerns pharmaceutical compositions comprising DAPK or inhibitors or activators thereof in the treatment, prevention, amelioration and delay of cancer or in the induction or suppression of inflammation as well as predictive methods for determining the risk of developing cancer in a subject, for determining the overall and disease-free survival time of a subject suffering from cancer and for deciding a personalized therapy for a subject suffering from cancer. Further, kits in order to perform said predictive methods are described.

## Description

### Technical Field

The present invention concerns pharmaceutical compositions comprising DAPK or inhibitors or activators thereof in the treatment, prevention, amelioration and delay of cancer or in the induction or suppression of inflammation as well as predictive methods for determining the risk of developing cancer in a subject, for determining the overall and disease-free survival time of a subject suffering from cancer and for deciding a personalized therapy for a subject suffering from cancer. Further, kits in order to perform said predictive methods are described.

### Background of the invention

Death-associated protein kinase 1, herein shortly named as DAPK, is an enzyme that in humans is encoded by the DAPK1 gene. Death-associated protein kinase 1 is a positive mediator of gamma-interferon induced programmed cell death. DAPK1 encodes a structurally unique 160-kD calmodulin dependent serine-threonine kinase that carries 8 ankyrin repeats and 2 putative P-loop consensus sites.

The Death-Associated Protein kinase (DAPK) family contains three closely related serine/threonine kinases, named DAPk, ZIPk and DRP-1, which display a high degree of homology in their catalytic domains. The Death associated protein kinase (DAPK) is a calcium/calmodulin-regulated serine/threonine kinase with a protective role during chronic inflammation in UC and UCC (UC-associated carcinoma). ⁶⁸Interestingly, DAPK can regulate inflammation either positively through NLRP3 inflammasome formation⁶⁹ or negatively through inhibition of NFκB.^{70,71} As inflammation compromises gut homeostasis and is also associated with cancer progression,⁷² it is important to understand the role of key molecules that are involved in the activation of the inflammatory cascade.

### DAPK as a Tumor Suppressor

An increasing amount of evidence supports the idea that DAPK works as a tumor suppressor gene in *vivo* inhibiting metastasis (Inbal et al. 2000). It was first noticed that DAPK mRNA and protein expression are lost in various types of cancer cell lines. Over-expression of DAPK induces apoptosis, whereas loss of its function leads to protection against programmed cell death (Cohen et al. 1997). These studies have been published showing DAPK promoter hypermethylation and consequent expression silencing in tumors that had been freshly isolated in patients. A repressed expression of DAPK by hypermethylation in the promoter CpG region of gene has been shown for various human cancers. It has been suggested that DAPK promoter hypermethylation may play an important role in the early step of tumor progression of colorectal carcinoma (Mittag et al., 2006). In long-standing ulcerative colitis (UC) and ulcerative colitis-associated carcinoma (UCC), promoter methylation correlated significantly with decreased DAPK protein expression and severity of inflammatory activity. All together, these data well establish the tumor- and metastasis-suppressive functions of DAPK, explaining why this gene is subjected to an activation or loss during tumor development.

WO 98/58052 describes DAPK isolation and alleges its therapeutic implication in cancers or immune disorders where DAPK is being expressed or where there is a decreased expression of DAPK. It is suggested to provide a composition for administering DAPK or a fragment or a derivative thereof to a subject to prevent or treat cancer which is associated with decreased expression of DAPK. Further, an agonist specific for DAPK is suggested to be administered to a subject to prevent or treat cancer. In a further embodiment, antagonists which decrease the expression and activity of DAPK are suggested to be administered to a subject to prevent or treat cancer which is associated with increased expression of DAPK. However, WO 98/58052 is completely silent in providing any experimental data supporting these mere allegations. Instead of providing any facts supported by research data, WO 98/58052 provides a laundry list of cancers which may be prevented or treated by administering DAPK, agonists thereof and antagonists thereof without any proof of this concept.

WO 2012/081017 provides compositions and methods for treating cancer and neurodegenerative diseases and discloses agents useful in regulating DAPK activity. It is stated that promoting DAPK activation is beneficial in the treatment of proliferative diseases such as cancer while inhibiting or reducing DAPK activity is considered as beneficial in the treatment of pathologies associated with neuronal cell death.

WO 2009/081564 provides a medicine effective in preventing and/or treating illness concomitant with cell death from stroke by inhibiting DAPK. It is described that inhibition of DAPK would likely intercept cell death and prevent further damage of ischemic regions in cerebral infarction and other ischemic diseases.

EP 0 981 610 describes that the absence of DAPK leads to a pre-disposition for cancer and/or metastasis and that DAPK promotes death of normal or tumor cells and suppresses the metastatic activity of tumor cells.

Summarily, DAPK is known in the art to play an important role in programmed cell death and inhibiting its expression or neutralization of its expression products protects the cell from cell death. This is in line with the concept of DAPK acting as a tumor suppressor gene.

### Alternative Splicing of DAPK

Death-associated protein kinase (DAPK) is undergoing an alternative splicing leading to the production of two isoforms with antagonistic functions (Jin and Gallagher, 2003). These two forms differ in 10 amino acids (RNSHVWNPTV) at the C-terminus of the pre-mRNA and have antagonistic functions: DAPKalpha possesses pro-apoptotic function whereas DAPKbeta attenuates TNF-induced apoptosis (Jin et al., 2006). The extra amino acids residues on the carboxyl-terminus of the DAPK pre-mRNA could be interpreted as intron retention, a subtype of alternative splicing. However, the exact mechanism of DAPK pre-mRNA alternative splicing remains unknown. In the present application, the abbreviation "DAPK" is used for a mixture of both isoforms wherein the proportion of each isoform in said mixture is not determined.

### Problem of the invention

It is a problem underlying the present invention to provide new pharmaceutical compositions in order to treat, prevent, delay or ameliorate cancer or inflammation or in order to induce inflammation. Further, there is a need for providing predictive methods for determining the risk of developing cancer, of determining the overall survival time of a subject suffering from a cancer and for methods for designing personalized therapy for a subject suffering from a cancer. Further, kits for performing said predictive methods are disclosed.

### Summary of the Invention

The present invention provides compositions for modulating DAPK activity in order to prevent or delay or ameliorate or treat cancer and in order to induce or suppress inflammation. In addition, in vitro methods are provided which can be used in the field of prospective medicine in order to determine the risk of developing cancer and the overall survival time of a subject suffering from a cancer and for designing a personalized therapy for a subject suffering from a cancer by evaluating the expression levels of DAPK. In a more general way, the term "treating" is used not only for curing a disease like cancer or inflammation but comprises also ameliorating, delaying and preventing said diseases.

According to the present and established teaching in science, DAPK acts a tumor suppressor which is mainly expressed in normal cells while in tumor cells or in cells associated with tumors or inflammation, a significant decrease of DAPK protein expression has been found which is also correlated with higher inflammatory activity. Contrary to this established teaching in the art, according to one aspect of the invention the present inventors have surprisingly found that inhibition of DAPK, i.e. a supposed tumor suppressor, can be used efficiently to ameliorate, delay, prevent or treat cancer. Further, inhibition of DAPK, i.e. a lower expression of DAPK in a cell or in a tissue could be positively correlated with a higher survival rate of patients, specifically those suffering from a colon cancer, ulcerative colitis-associated carcinoma (UCC) or ulcerative colitis (UC) which tends under certain conditions to change over to ulcerative colitis-associated carcinoma (UCC). This was highly surprising and could not have been expected from the prior art describing tumor suppressor activity of DAPK.

It is also disclosed herein for the first time that the isoforms of DAPK, DAPKα and DAPKβ, play a crucial role in cancer and cancer development. It has been found that cells with a high grade of inflammation as well as cancer cells show a reduced or no expression of DAPKβ isoform while, on the other hand, inflammation negative cells or tissues, or cells or tissues with a lower grade of inflammation as well as normal cells show a remarkably higher expression of DAPKß. This finding offers the possibility to provide more tailored and specific pharmaceutical composition distinguishing between activators and inhibitors of DAPKα and DAPKβ. Also in the field of prospective medicine more specific assays can be provided based on the finding that DAPKβ isoform is expressed in a lowamount in pre-neoplastic regions and carcinomas while normal cells and tissues show a remarkably higher expression of DAPKβ. This finding is particularly true for colon epithelial cells but also for immune cells.

According to the first aspect of the invention, a pharmaceutical composition is provided which comprises a therapeutically effective amount of an inhibitor of DAPK and a pharmaceutically acceptable carrier which is used in a method of treating or preventing or delaying or ameliorating cancer. As explained above, "DAPK" is to be understood as a mixture of both isoforms DAPKα and DAPKβ, where the proportion of presence of DAPKα and DAPKβ in said mixture has not been determined.

According to a further aspect of the invention, a pharmaceutical composition is provided which comprises a therapeutically effective amount of an activator of DAPK and a pharmaceutically acceptable carrier which is used in a method of treating or preventing or delaying or ameliorating cancer.

According to an additional aspect of the invention, a pharmaceutical composition is provided comprising a therapeutically effective amount of an inhibitor or an activator of DAPK in order to induce inflammation.

According to a further additional aspect, the present invention provides an in vitro method for determining the risk of developing cancer in a subject comprising determining the expression level of DAPK.

According to a further aspect of the present invention, an in vitro method for determining the overall survival time of a subject suffering from a cancer comprising determining the expression level of DAPK is described.

In a still further aspect of the present invention, an in vitro method for designing a personalized therapy for a subject suffering from cancer comprising determining the expression level of DAPK is provided.

According to other aspects of the invention, kits comprising means are provided in order to detect the presence and the amount of DAPK in a sample of a subject supposed to suffer from cancer in order to perform the in vitro methods for designing a personalized therapy, for determining the overall survival and disease-free time of a subject suffering from a cancer and for determining the risk of developing cancer in a subject.

According to another aspect, the present invention provides a pharmaceutical composition comprising a therapeutically effective amount of DAPK or an activator of DAPK together with a pharmaceutically acceptable carrier in order to suppress inflammation.

All above described aspects involve embodiments wherein DAPK is distinguished between DAPKalpha and DAPKbeta providing different effects and properties of the embodiments of the invention.

It is understood that the foregoing detailed description and the following examples are illustrative only and are not to be taken as limitations upon the scope of the invention. Various changes and modifications to the disclosed embodiments, which will be apparent to those of skill in the art, may be made without departing from the spirit and scope of the present invention. Further, all patents, patent applications, and publications identified are expressly incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents are based on the information available to the applicants and do not constitute any admission as to the correctness of the dates or contents of these documents. All prior art recited herein is incorporated by reference.

### Brief Description of the Drawings

**Figure** 1
   DAPK, pSTAT3Y705, and TNF expression pattern in IEC of normal mucosa, UC and UCC. Tissue sections derived from control and different stages of UC patients were analyzed by IHC. The pSTAT3Y705 expression was predominantly nuclear. Representative images of (A1-A5) HE, (B1-B5) DAPK, (C1-C5) pSTAT3Y705 and (D1-D5) TNF staining in non-IBD (n=11), inactive UC (n=49), low-active UC (n=41), highly-active UC (n=15) and UCC (n=24) are shown. Original magnification: 200x.
Figure 2
   Vertical scatter plots of (A) DAPK, (B) pSTAT3Y705, and (C) TNF scores grouped by sample type (non-IBD, inactive UC, low-active UC, highly active-UC and UCC). A non-parametric Mann Whitney test was performed to evaluate significance between two sample groups as depicted (where p≤0.05) above the scatter plot.
**Figure 3**
   (A) DAPK and pSTAT3Y705expression pattern in DALM. Representative images of DAPK and pSTAT3Y705 staining in DALM tissue sections (n=11) depicting heterogeneous pattern of expression are shown. Areas with high DAPK-high pSTAT3Y705 (►); high DAPK-low pSTAT3Y705 (A); low DAPK-high pSTAT3Y705 (√) and low DAPK-low pSTAT3Y705 (→) expression are indicated by arrows. Note the serial sections for DAPK and pSTAT3Y705 allowing direct comparison of expression in the same region of interest. TNF-induced functions in HCEC cells - HCEC cells were stimulated with 0.66 ng/ml of TNF for various time points (1, 6, 24, 48, or 72 hours). (B) DAPK and (C) STAT3 expression/activation were assessed by immunoblotting using the corresponding antibodies. Representative Western blots of five independent experiments are shown. (D) IL-6 and (E) IL-8 secretion was measured in cell culture supernatants by ELISA. Data were obtained from more than five i 720 independent experiments performed in duplicates or triplicates. *p<0.05 vs untreated control cells.
**Figure 4**
   Epression/activation of DAPK/STAT3 through intestinal inflammation in the AOM+DSS-induced mouse model of CAC. (A) Scheme for the experimental course inducing Colitis-associated tumors in wild-type mice by AOM (10mg/kg) and 3 cycles of DSS (2.5%). Endoscopic images display (B) mouse colon before treatment; (C) after 7 days of DSS (2.5%) in drinking water; (D) before the start of the 2nd cycle of DSS application at day 22 and (E) before tumor harvest at the end of the protocol at day 65. (F) DAPK and (G) pSTAT3Y705 expression/activation was analyzed by Western blotting of tissue extracts from colon of control (n=2), DSS-treated (n=4) and AOM+DSS-treated (n=5) mice using the corresponding antibodies.
**Figure 5**
   DAPK attenuates TNF-induced IL-6 secretion and STAT3Y705 phosphorylation in HCEC cells. (A,C,E) HCEC cells were treated with 0.66 ng/ml of TNF for 24 and 48 hours in the presence (DAPK- or non-specific siRNA) or absence of siRNA. (A) DAPK knockdown and STAT3Y705 phosphorylation was assessed by Western blotting of whole cell lysates. (C) IL-6 mRNA expression was analysed by real-time RT-PCR. Two similar experiments were performed. +p<0.05 vs respective control; *p<0.05. (E) IL-6 was measured in cell culture supernatants by using an ELISA. Three similar experiments were performed in duplicates or quadruplicates. +p<0.05 vs respective controls; *p<0.001. (B,D) HCEC cells were treated with 0.66 ng/ml TNF for 6, 24, 48, or 72 hours in the presence or absence of 10 µM DAPK inhibitor. (B) DAPK and pSTAT3Y705 expression was analyzed by Western blotting. (D) IL-6 was measured in cell culture supernatants by using an ELISA. Two independent experiments were measured in triplicates. +p<0.001 and #p<0.001 vs TNF ± DAPK inhibitor treatment; *p<0.05 (F) TNF-induced DAPK/STAT3 complex formation in HCEC cells. HCEC cells were treated with 0.66 ng/ml 746 TNF for 6, 24, 48, or 72 hours. DAPK was immunoprecipitated and complexes were transferred to nitrocellulose membrane. The membranes were probed with anti-STAT3 and anti-DAPK antibodies. Blots from a representative experiment (n=2) are shown.
**Figure 6**
   Structural analysis of the DAPK-STAT3 complex. (A) Docked complex of STAT3-STAT3 dimer. (B) Docked complex of DAPK to the STAT3 dimer. (C) Superposed view of DAPK and JAK binding to the monomer of STAT3. DAPK (PDB ID: 1JKS) is represented in red, STAT3 (PDB ID: 1BG1) is represented in blue and the JAK (PDB ID: 3EYG) is represented in green.
**Figure 7**
   TNF induces STAT3 nuclear translocation and DNA binding. HCEC cells were treated with 0.66 ng/ml TNF for 24, 48, or 72 hours. (A) Cytoplasmic and nuclear proteins were separated to analyze STAT3 distribution by immunoblotting with anti-pSTAT3Y705, anti-STAT3, and anti-histone H1/anti-GAPDH antibodies. Western blots are representative of three independent experiments. (B) DNA binding activity of STAT3 in nuclear extracts was evaluated by non-radioactive EMSA using infrared labeled oligonucleotides containing the STAT3 consensus sequence in the presence or absence of 100x competitor (Com) or mutant (Mut) oligos. Results are representative of two independent experiments. (C) Theoretical picture of the DAPK promoter showing STAT3 binding motifs distributed in two regions. (D,E) HCEC cells were treated with 0.66 ng/ml TNF for 48 hours. The ChIP assay was performed by using IgG or anti-pSTAT3Y705 antibodies. (D) qPCR and (E) end point PCR followed by agarose gel electrophoresis were used to analyze STAT3 binding to both regions of the DAPK promoter. Data are derived from four independent experiments performed in duplicates. *p<0.05 vs the corresponding control, Mann-Whitney U-test.
**Figure 8**
   STAT3 regulates the expression of DAPK. (A) HCEC cells were treated with 0.66 ng/ml TNF for 24, 48, or 72 hours in the presence or absence of 20 µM AG490. pSTAT3Y705 and DAPK expression were assessed by 772 immunoblotting (upper panel). DAPK mRNA expression was analyzed by real-time RT-PCR. Data represent the levels of DAPK mRNA after normalization to β2-microglobulin levels and expressed relative to the value of the untreated controls. Results were obtained from two independent experiments performed in duplicates. +p<0.05 vs untreated control; #p<0.001 vs 20 µM AG490 treated cells; *p<.001 (lower panel). (B) HCEC cells were treated with 0.66 ng/ml TNF for 24, 48, and 72 hours in the presence or absence of 7 µM Stattic. pSTAT3Y705 and DAPK expression were assessed by immunoblotting of whole cell lysates (upper panel). DAPK mRNA expression was analyzed by real-time RT-PCR. Data represent the levels of DAPK mRNA after normalization to β2-microglobulin levels and expressed relative to the value of TNF treatment at the corresponding time point. Two similar experiments were performed, each in duplicate. *p<.001 (lower panel). (C) Working model depicting TNF-induced signaling and functions in HCEC. TNF induces a dual signaling pro-inflammatory IL-6→STAT3 and anti-inflammatory-DAPK pathways. See the text for more details. Arrows depict pro-inflammatory signaling of TNF and dashed lines show anti-inflammatory signaling of TNF.
**Figure 9**
   **A.** PCR amplification of DAPK-α (698bp) and DAPK-β (225bp) alternative spliced isoforms in colon cancer HCT116 and normal colon epithelial (HCEC) cells upon TNF stimulation (0.66ng/ml).
   **B.** Whole HCT116 and HCEC cell lysate was subjected to western blot and immunoprobed with specific DAPK antibody.
   **C.** Western blot analysis of not-differentiated macrophages (o), differentiated (stimulated by 16 nM PMA for 72h) macrophages and stably transfected U937T with DAPKα or DAPKβ constructs.
   **D.** Histograms of FACS analysis of Annexin-V/Propidium iodide staining of untreated (U937T) and transfected with either DAPKα or DAPKβ cDNA U937T cells. Note that the level of apoptosis (the sum of the early apoptotic cells (lower right segment) and the late apoptotic cells (upper right segment in each quadrat)) in control is 13%, transfected with DAPKα is 38.6%, transfected with DAPKβ is 17.9% (lower panel).
**Figure 10**
   **A.** Representative agarose gel indicated DAPKα or DAPKβ alternatively spliced isoforms, amplified by RT-PCR from the human tissue microarray. CA - carcinoma; LG - low grade of inflammation; NC - negative control (no template); PC - positive control (HCT116 cell lysate).
   B. RT-PCR using specific primers for DAPK from two patients with different tissues areas: LG - low grade of inflammation, HG - high grade of inflammation, DS - dysplasia, CA - carcinoma.C.
**Figure 11**
   Western Blotting of AOM-DSS-tumors of mice treated with the DAPK-inhibitor (number 1-3) and untreated control mice (number 4, 5: 4.1-4.3 and 5.1-5.3 represent different colorectal regions of the mice where the tumors originated from). The NFκB inhibitor plκß was up-regulated in tumors from mice treated with the DAPK-inhibitor, leading to its degradation and thus to the shuttling of NFκB to the nucleus. This will finally induce an inflammation-promoting pathway. In parallel a downstream target of DAPK, the tumor suppressor p53, was down-regulated after DAPK-inhibitor treatment.
**Figure 12**
   Endoscopic images display AOM-DSS tumors of different size (left: without DAPK-inhibitor; right: with DAPK-inhibitor). There were less and smaller tumors in the gut from mice treated with the DAPK-inhibitor.
**Figure 13**
   Evaluation of tumor loading after complete resection of the gut by macroscopically counting the number of tumors (A). Immunohistochemical evaluation of tumors from formalin-fixed in paraffin embedded tissue sections. Tumors arising in untreated control mice showed a rather non-invasive growth, low grade atypia, and a simple and tubular architecture. Tumors from mice treated with the DAPK-inhibitor were invasively growing, showed high grade atypia, and a complex, cribiform architecture (B).
**Figure 14**
   Evaluation of DAPK expression pattern in normal mucosa, UC (ulcerative colitis) and UCC (UC-associated carcinoma) human tissue specimens. Tissue sections derived from control and different stages of UC patients were analysed by immunohistochemistry. The DAPK protein expression was cytoplasmic. Representative images of HE-staining and DAPK-staining in non-IBD (n=11), inactive UC (n=49), low-active UC (n=41), highly-active UC (n=15) and UCC (n=24) are shown. Original magnification: 200x (A) Vertical scatter plot of DAPK scores grouped by sample type (non-IBD, inactive UC, low-active UC, highly-active UC and UCC) (B). A non-parametric Mann Whitney test was performed to evaluate significance between two sample groups as depicted (where p≤0.05) above the scatter plot.
**Figure 15**
   Kaplan-Meier-curve for DAPK and survival time (months) in patients with colorectal cancer. DAPK overexpression was significantly correlated with shorter overall survival (p<0.001, log rank test).

### Detailed Description of the invention

The present invention provides a pharmaceutical composition comprising a therapeutically effective amount of an inhibitor of DAPK and a pharmaceutical acceptable carrier for use in a method of treating or preventing or ameliorating or delaying cancer. The inhibitors of DAPK which can be used according to the present invention are either known per se or can be found by well-established and known methods for screening of an inhibitor.

In one embodiment of the invention, a method for screening of an inhibitor effective in treating or preventing or ameliorating cancer is provided, comprising the steps of
(a) exposing a cell expressing DAPK to a putative inhibitor;
(b) determining inhibition of DAPK;
wherein inhibition of DAPK indicates that said inhibitor is effective in treating or preventing or delaying or ameliorating cancer.

The methods for screening of an inhibitor may be for instance high-throughput screening essays. Cells expressing DAPK may be brought in contact with a candidate inhibitor which can be done either in vivo or in vitro. The cells or extracts thereof, for instance protein extracts of said cells, are then assayed in order to detect DAPK which is inhibited.

In one embodiment of the invention, said inhibitor of DAPK is a compound of formula (1), 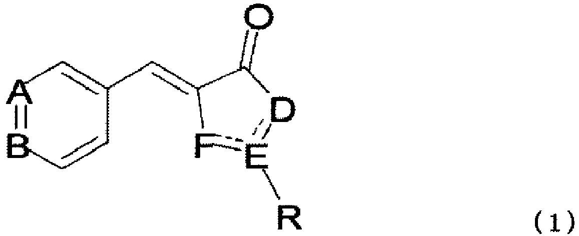
in which one of A and B represents a nitrogen atom and the other a carbon atom,
D, E and F represent the combination O-C=N or N=C-S,
and R represents a substituted or unsubstituted, linear, branched or cyclic, saturated or unsaturated lower alkyl group or aralkyl group,
a substituted or unsubstituted aryl group, or a linear, branched or cyclic, substituted or unsubstituted, saturated or unsaturated aminoalkylgroup,
preferably wherein R represents a linear, branched or cyclic, optionally substituted lower alkyl group, an optionally substituted saturated or unsaturated aralkyl group, an optionally substituted aryl group, or a linear, branched or cyclic substituted or unsubstituted aminoalkylgroup, particularly preferably wherein R represents a linear, branched or cyclic lower alkylgroup, a saturated or unsaturated lower aralykyl group, a linear, branched or cyclic aminoalkyl group or a group of formula (2), 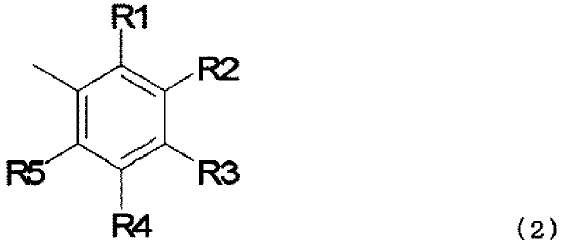
in which R1 to R5 independently represent a hydrogen atom, a halogen atom, a nitro group, a lower alkyl group or a lower alkoxy group;
or a salt, solvate, hydrate, active metabolite,prodrug or derivatives thereof.

"Lower" in regard to alkyl group means that the carbon number thereof is between 1 and 15, preferably between 1 and 10, particularly preferably between 1 and 4. In regard to aralkyl groups the term "lower" refers to the alkyl part of the group and means a carbon number between 1 and 15, preferably between 1 and 10, particularly preferably between 1 and 4.

Regarding R1 to R5, the term "lower" in reference to alkyl group or alkoxy group means a carbon number of 1 to 6, preferably of 1 to 3.

Halogen within this description refers to fluorine, chlorine, bromine and/or iodine

An alkyl group refers to a carbon chain, an aralkyl group refers to an alkyl group wherein one or more hydrogen atoms are substituted by an aryl group, and an aryl group refers to a functional group derived from an aromatic ring. A preferable aryl residue in the aryl group or aralkyl group is a phenyl residue.

A cyclic group can be monocyclic or polycyclic, e.g. also encompass di- or tricyclic groups apart from monocyclic groups. An exemplary polycyclic group is e.g. an adamantly group.

Suitable substituents for the alkyl, aryl, aralkyl and aminoalkyl groups include one or more of halogen atoms, a nitro group, a lower alkoxy group and/or an amine group preferably one or more of halogen atoms, a nitro group or a lower alkoxy group.

Preferable compounds as DAPK inhibitors in the present invention are compounds of the following formulas: 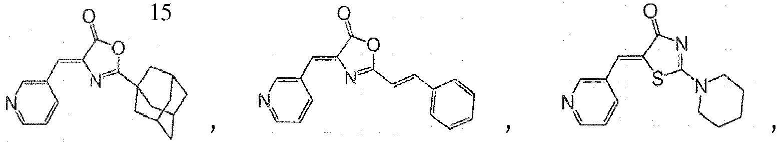 and compounds of formulas (3) and (4).

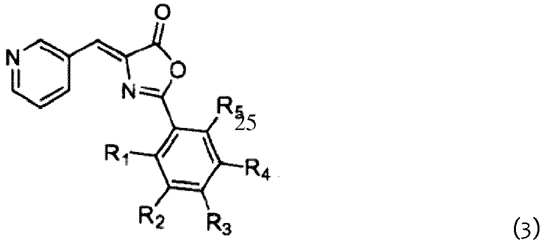

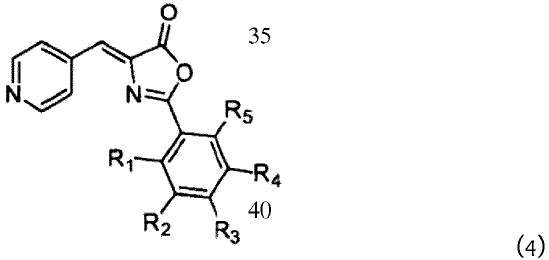

Preferable compounds of formulas (3) and (4) include

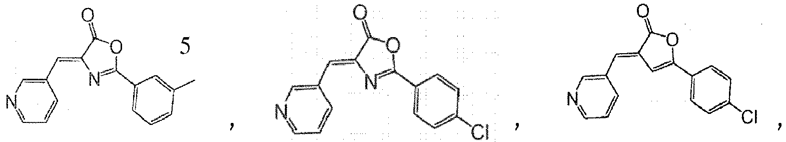

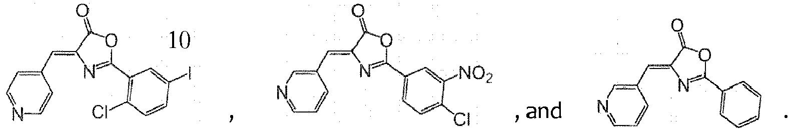

Particularly preferred among these compounds is

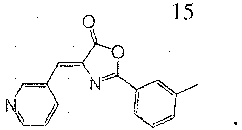

In another embodiment a suitable compound is

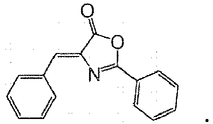

Further compounds may be tested to be effective as an inhibitor. More detailed information on how to perform screening assays for inhibitors but also for activators of DAPK are included in the present application. Also with respect to the formulation or pharmaceutical composition, the therapeutically effective amount of the agent as well as the pharmaceutically acceptable carriers and the administration modes of the presently described pharmaceutical compositions, the corresponding amounts, specific compositions etc., may be tested by the skilled artisan by routine laboratory experiments in order to determine those compositions which are most useful for performing the present invention.

As discussed already above, the presently claimed method of treatment using the said composition is contrary to the teaching in the art wherein DAPK is described as tumor suppressor wherein up-regulation of DAPK provides a benefit in avoiding, ameliorating or treating cancers. The inventors, however, have found that DAPK up-regulation provides an adverse prognostic factor in the development of cancer, particularly colon cancer.

In order to investigate if the immunohistochemical DAPK protein expression is associated with colon cancer progression and prognosis the inventors analyzed samples from 220 colorectal cancer patients. The formalin-fixed paraffin embedded tissues were spotted in quadruplicates (2 spots from the tumor center, 2 spots from the invasion front) on tissue microarrays (TMA) and were immunohistochemically stained for DAPK. DAPK loss was more frequently found in G1/G2 tumors, in left-sided tumors, in No and Mo tumors, and in tumors without lymphatic and vascular invasion (Table 1).

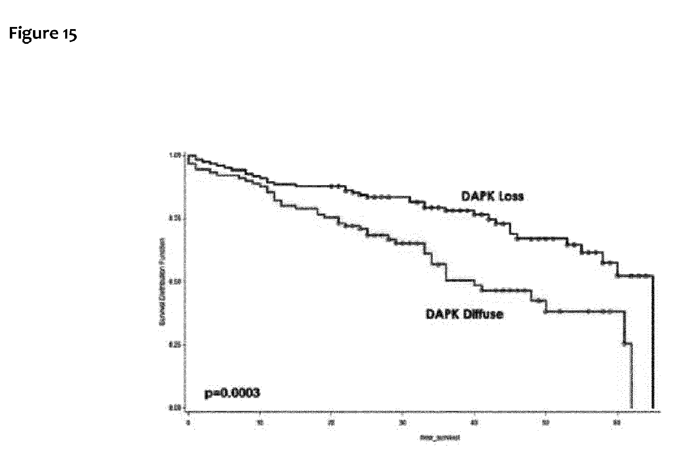

In Kaplan-Meier-curve and log-rank analysis, DAPK overexpression was associated with shorter overall survival time (p<0.001; Figure 15). In patients with DAPK negative tumors, the 5-year survival rate was 52.3% whereas it was only 25.6% in patients having DAPK positive tumors. In parallel, DAPK positive tumors showed significant lower number of tumor infiltrating lymphocytes (iTIL and sTIL) than DAPK negative tumors (p=0.01; Table 1). Interestingly in 76.1% of DAPK positive tumors signs of tumor budding were observed (p<0.001; Table 1). According to literature a high rate of tumor infiltrating lymphocytes (iTIL and sTIL) is beneficial for patients' outcome whereas signs of tumor budding are correlated with tumor aggressiveness and worst clinical prognosis.

When all classical prognostic factors such as pT, pN and pM stage were included in a multivariate Cox regression analysis, DAPK retained its strong independent prognostic value together with pN and M (Table 2, p<0.01). Patients with DAPK overexpression tumors had a 1.9 times greater risk of death in comparison to patients with DAPK negative tumors, when adjusting for the prognostic effects of the remaining features (pT, pN, and pM).

Based on this finding, the advantage of administering a DAPK inhibitor in order to treat, ameliorate, delay or prevent cancer have been found. Inhibiting DAPK will, therefore, provide a higher survival time for patients who received a therapeutically effective amount of a DAPK inhibitor; these patients will benefit in the form of prevention, delay, amelioration or therapy of cancer. Specifically, colon cancers can be treated by this therapy. Further, ulcerative colitis (UC) and ulcerative colitis-associated carcinoma (UCC), gastro-intestinal tract cancers as well as colorectal cancers may be treated successfully.

The present invention can also be used in the field of predictive medicine. According to the enormous economic pressure put on health insurance companies and the public health service, methods for determining the risk of developing cancer, for determining the overall or disease-free survival time of a subject as well as designing personalized therapies for a subject suffering from cancer are needed. This problem is solved according to the invention by the methods described in the following.

First of all, an in vitro method for determining the risk of developing cancer in a subject is provided. The expression level of DAPK in a sample potentially associated with cancer of said subject is determined. In those samples of patients, where the expression level of DAPK is increased with respect to a reference value, said subject has an increased risk of developing cancer. In those cases where there is a decrease with respect to a reference value, said subject has a decreased risk of developing cancer.

As already described above, the inventors have surprisingly found that DAPKalpha and DAPKbeta are expressed differently in tumor cells (=carcinoma cells), cells under inflammatory conditions as well as normal "healthy" cells and tissues thereof. "DAPK" is to be understood to comprise both DAPKalpha and DAPKbeta, i.e. a combination of both isoforms. An increase in DAPKalpha with respect to a reference value stands for an increased risk of developing cancer while a decrease in DAPKalpha stands for a decreased risk of developing cancer. Vice versa, an increase of DAPKbeta stands for a decreased risk of developing cancer while a decrease in DAPKbeta stands for an increased risk of developing cancer. "Increased level" and "decreased level" have to be understood to be compared to a reference figure of a normal, not transformed or pathologically changed cell or tissue.

In a further embodiment of the invention, an in vitro method for determining the risk of developing cancer in a subject is provided. The expression level of DAPK, specifically DAPKbeta, is determined in a sample which is potentially associated with cancer of said subject. If the expression level of DAPK, specifically DAPKbeta, is increased with respect to a reference value, said subject has a decreased risk of developing cancer while in those cases where the expression level of DAPKbeta is decreased with respect to a reference value, said subject has an increased risk of developing cancer.

Similarly, the pharmaceutical composition described above comprising an inhibitor of DAPK may further specifically comprise an inhibitor either of DAPKalpha or DAPKbeta. An inhibitor of DAPKalpha causes either an absolute or at least a relative increase of DAPKbeta in relation to the amount of DAPKalpha.

The experimental evidence for this therapeutic implication is given in the following.

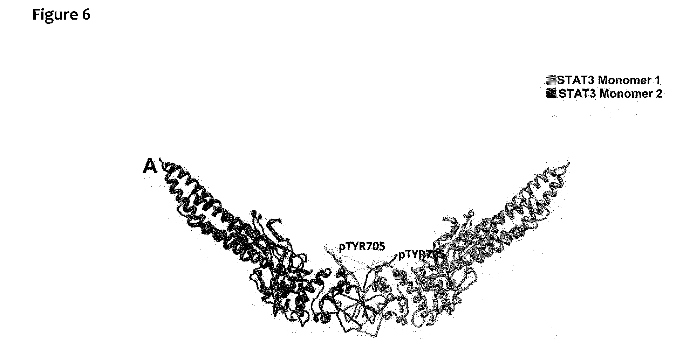

The inventors treated normal intestinal epithelial cells (HCEC) and tumor cells (HCT116) with 0.66 ng/ml of TNF and harvested them at 6, 24 and 48 hours. Using sequence-specific primers it could be shown by RT-PCR that untreated HCT116 cells predominantly expressed α isoform promoting pro-apoptotic function of DAPK, whereas in HCEC cells a switch from the α to the β isoform was detected under continuous inflammatory conditions (Figure 6A). Moreover, in native normal cells DAPKβ is the dominant isoform. In tumor cells DAPK β mRNA induction was not observed during TNF treatment. This cell-specific reaction could explain, at least partially, the different response of normal and malignant epithelial cells to TNF: DAPK activation forces tumor cells to caspase-3 - dependent cell death (Bajbouj et al., 2009). Otherwise TNF stress in HCEC cells directs them to an inflammatory pathway, mediated by a novel DAPK/STAT3 complex as has been found by the present inventors.

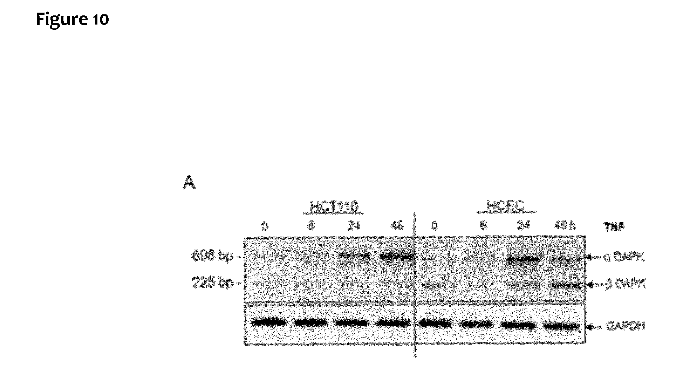

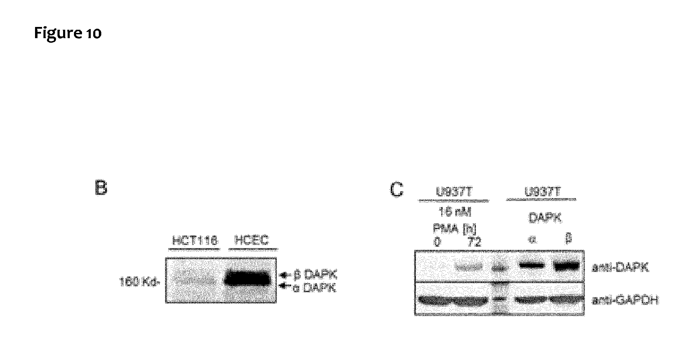

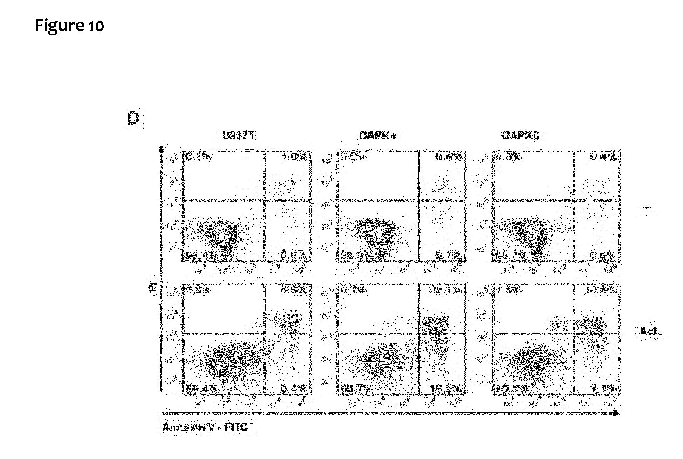

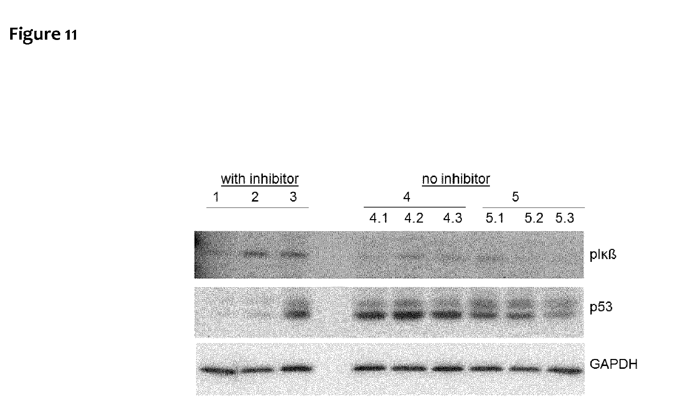

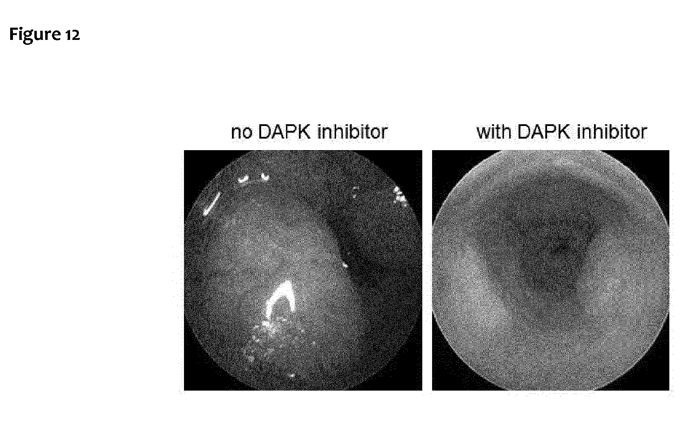

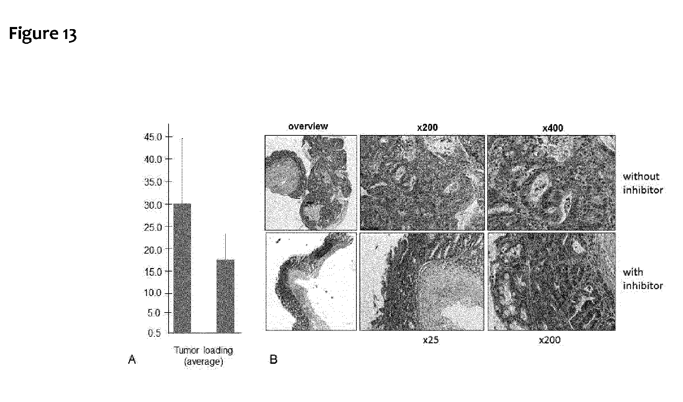

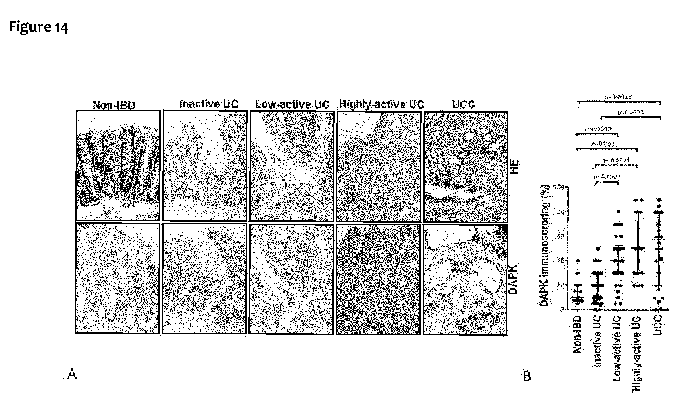

To the inventors' knowledge, there is no antibody specific for the DAPKβ protein available up to now. In order to confirm the presence of different DAPK isoforms on the protein level, the inventors employed an overnight SDS-PAA gel running system. This allowed separating the DAPKβ isoform which can be detected by the DAPK antibody epitopes common for both isoforms (BD Biosciences) (Figure 10B).

Based on the pRK5F-DAPKα-Flag plasmid (kindly provided by Prof. R.H. Chen, Taiwan National University) a pRK5F-DAPKβ-Flag construct was generated. To verify the full performance of constructs for further investigations, we tested them in a macrophage cell line. U937T cells were stably transfected by electroporation with linearized 10 µg either DAPKα or DAPKβ cDNA, followed by single cell sorting after one week selection for the CD8 T-cell marker. The clones were expanded and immuno-probed against the DAPK-specific antibody (Figure 10C). Loading control was indicated by GAPDH antibody. DAPKα over-expression in differentiated macrophages (treated with 16mM PMA) led to a significant increase (up to 3 fold) in apoptosis whereas DAPKβ over-transfection does not enforce cell death, thus confirming the previously reported (Jin et al., 2003) pro-survival function (Figure 6D).

The inventors investigated whether different isoforms of DAPK exist in human tissues from patients with ulcerative colitis (UC) or UC-associated tumors. The inventors designed a tissue microarray (TMA) of UC-associated tissues with different grades of inflammation as well as preneoplastic regions and carcinomas. The TMA contained 49 formalin-fixed paraffin-embedded tissue samples from 11 patients. A 5-µm thick section was cut from each paraffin block and the histological mirror image on an HE slice has been evaluated. RNA was extracted using a technique based on proteinase K digestion and magnetic beads precipitation.

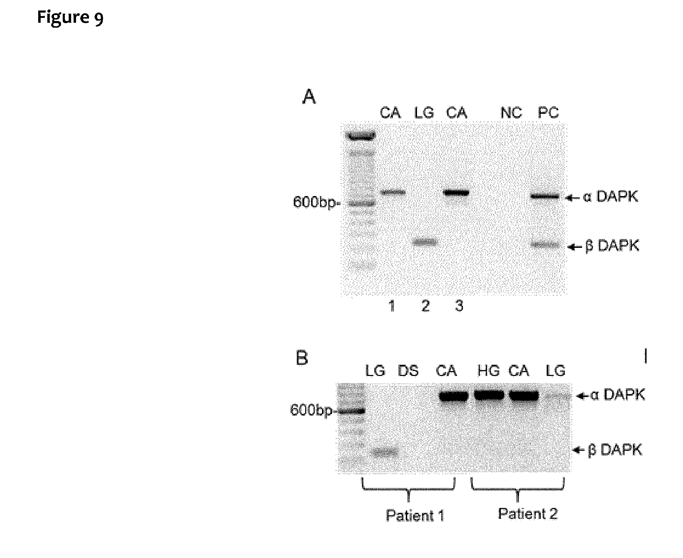

In samples where DAPK mRNA was present, the inventors observed a very interesting tendency: the samples with low grade of inflammation showing only DAPKß variant (3 out of 8). One sample from these eight expressed both, but profound DAPKß isoform. High grade inflammation and cancer areas never showed the DAPKß isoform. Moreover, different regions from the same patient indicated the expression of different DAPK isoforms suggesting a possible correlation between the grade of inflammation and cell specificity in DAPK alternative splicing regulation. Representative examples of obtained amplicons are indicated on agarose gels in Figure 9A and 9B.

Summarily, the expression of DAPKbeta protein, specifically in epithelial cells and/or in immune cells, is an important cancer prognostic marker and provides as well therapeutic methods in order to treat cancer diseases. Activation of DAPKbeta is beneficial for treating tumor cells including the amelioration, preventing and delaying of tumors.

Alternative splicing is a mechanism by which a single gene gives rise to several different proteins. Alternative splicing is accomplished by the concerted action of a variety of different proteins, termed "alternative splicing regulatory proteins," that associate with the pre-mRNA in the cell nucleus, and cause distinct alternative exons to be included in the mature mRNA. These alternative forms of the gene's transcript give rise to distinct isoforms of the specified protein like for DAPK comprising DAPKalpha and DAPKbeta. One option to modulate the amount of DAPKalpha and DAPKbeta, respectively, is, therefore, to influence the splicing machinery at DAPK pre-mRNA splicing and to use agents which selectively increase or decrease either DAPKalpha or DAPKbeta. Said agents can be found and selected by the skilled artisan. Corresponding methods are encompassed by the present invention. One exemplary method is described briefly in the following.

A method of modifying the activity of RNA alternative splicing regulatory proteins within cells comprising:
a) introducing into cells an agent, e.g. polynucleotide sequences capable of binding to RNA alternative splicing regulatory proteins;
b) binding within cells the said agents, e.g. polynucleotide sequences to the RNA alternative splicing regulatory proteins; and
c) modifying within cells the activity of the RNA alternative splicing regulatory proteins with said binding in order to selectively produce either DAPKalpha or DAPKbeta.

In a further embodiment of the invention, a pharmaceutical is provided which comprises a therapeutically effective amount of an activator of DAPK or a therapeutically effective amount of DAPK and a pharmaceutically acceptable carrier in order to be used in a method for treating or preventing or delaying or ameliorating cancer. As can be taken from the experimental data contained in the present application, the inventors have found a novel negative regulation principle between DAPK and STAT3, which balances TNF-induced inflammation. This has been particularly shown in colon carcinomas, specifically colitis-associated carcinoma and ulcerative colitis-associated carcinoma. DAPK comprises also, as described above, the isoforms DAPKalpha and DAPKbeta and their different way of action as described herein.

The inventors found that TNF is an activator of the inflammatory pathway and is involved in the pathogenesis of various inflammatory diseases like ulcerative colitis, Crohn's disease and rheumatoid arthritis. TNF activates NFkappaB while NFkappaB activates IL-6 cytokine release. That release of IL-6 cytokine activates Janus kinases (JAK) which themselves activate STAT3 and increase expression of IL-6. Activated STAT3 causes an increase of inflammation in a cell or tissue. Starting with this information, the inventors have found that inhibition of STAT3 increases TNF-induced expression of DAPK. By inhibiting STAT3, an inflammatory reaction is inhibited. Or stated in another way: activation of STAT3 represses expression of DAPK. Inhibition or inactivation or at least a decrease of DAPK activates STAT3 and an inflammatory reaction. Vice versa, an activation of DAPK inhibits STAT3 and inhibits, therefore, an inflammatory reaction induced by TNF.

The present inventors could now demonstrate that activation of DAPK is inhibiting an inflammation reaction induced by TNF. Therefore, pharmaceutical compositions containing an activator of DAPK can be used in a method of treatment, delaying, preventing or ameliorating tumor diseases (cancers) as well as inflammatory diseases. A typical activator of DAPK is TNF. Recently the inventors have shown that also histone deacetylase inhibitors activate DAPK (Gandesiri et al. 2012) Further activators can be found with screening methods as described herein.

The inventors have particularly found that DAPK plays an anti-inflammatory role in tumor transformation in inflammation-associated colon cancer, particularly in the development of UC and UCC.

In a further embodiment of the invention, an in vitro method is provided in order to determine the overall survival time of a subject suffering from a cancer. The method comprises determining the expression level of DAPK in a tumor sample of said subject, wherein if the expression level of DAPK is increased with respect to a reference value, said subject has a shorter survival time. If the expression level of DAPK is decreased with respect to a reference value, said subject has an increased survival time. "DAPK" is to be understood as a combination, i.e. the sum of DAPKalpha and DAPKbeta. In those cases, where DAPKalpha is increased, the subject has a shorter survival time and in those cases where DAPKalpha is decreased, the subject has an increased survival time.

In a still further embodiment of the invention, an in vitro method for determining the overall survival time of a subject suffering from a cancer is provided. The method comprises determination of the expression level of DAPKbeta in a tumor sample of a subject. If the expression level of DAPKbeta is increased with respect to a reference value, said subject has a higher survival time, in those cases where said expression level of DAPKbeta is decreased with respect to a reference value, said subject has a decreased survival time.

"Survival time" according the invention also includes disease-free survival, i.e the time till occurrence of metastasis or recurrence.

The present invention also provides an in vitro method for designing a personalized therapy for a subject suffering from cancer. "Personalized therapy" means that depending on the result of the prognostic test on survival time and/or risk of cancer development, a particular individualized therapeutic approach is selected in order to provide a treatment for the individual in need of said treatment which is as beneficial as possible. The invention comprises determination of the expression level of DAPK in a potential tumor sample of said subject. In those cases where the expression level of DAPK is increased with respect to a predetermined value which is to be understood as a reference value, a specific therapy directed to prevent and/or to treat said cancer is selected. Specifically, said DAPK is to be understood to be DAPKalpha which is a disadvantageous prognostic marker while in case of evidence for an increased level of DAPKbeta expression, which is a beneficial prognostic marker, another kind of therapeutic approach might be selected.

In a still further aspect of the invention, an in vitro method for designing a personalized therapy for a subject suffering from a cancer is provided. The expression level of DAPK in a tumor sample of said subject is determined. If the expression level of DAPK is decreased with respect to a reference value, then a therapy directed to prevent and/or treat said cancer is selected. Specifically, said DAPK is DAPKbeta.

Said cancer is particularly selected from colon cancer, colorectal cancer, gastro-intestinal tract cancer or ulcerative colitis-associated cancer.

The determination of the expression level comprises the determination of the mRNA level of said gene, the determination of the cDNA level or determining the protein level encoded by said gene.

Determination of the expression level may be carried out by quantitative PCR in situ hybridization or by cDNA array. Determination of the protein level is carried out preferably by western blot or immunohistochemistry.

Said therapy which is to be decided and which depends on the expression level of DAPK, DAPKalpha and DAPKbeta, respectively, may be a chemotherapy, radiotherapy, or gene therapy. Other kinds of therapeutic approaches might be taken. Said personalized therapy comprises a predictive or therapeutic response prediction, optionally also measurement as a biomarker the follow-up response to therapy.

In a further embodiment of the invention, kits are provided which comprise means for detection of the presence of the amount of DAPK, DAPKalpha and DAPKbeta in a sample of a subject supposed to suffer from cancer in order to perform the above described in vitro predictive methods.

### Diseases to be treated

Cancers that can be treated, ameliorated, delayed or prevented by the pharmaceutical compositions of the present invention include lung cancer, breast cancer, colon cancer, brain cancer, neuroblastoma, prostate cancer, melanoma, glioblastoma multiform, ovarian cancer, lymphoma, leukemia, melanoma, sarcoma, paraneoplasia, osteosarcoma, germinoma, glioma and mesothelioma. In one specific embodiment, the cancer is lung cancer, colon cancer, brain cancer, neuroblastoma, prostate cancer, melanoma, glioblastoma mutiform or ovarian cancer. In another specific embodiment, the cancer is lung cancer, breast cancer, colon cancer, brain cancer, neuroblastoma, prostate cancer, melanoma, glioblastoma multiform or ovarian cancer. In a preferred specific embodiment, the cancer is a colon cancer, colorectal cancer, gastro-intestinal tract cancer or ulcerative colitis-associated cancer.

In a further embodiment of the invention, inflammatory diseases can be treated, ameliorated, delayed or prevented by the pharmaceutical compositions of the present invention. Said inflammatory diseases particularly include ulceritis and ulcerative colitis.

It is to be noted that the cancers and inflammatory diseases described above are given only by the way of example and other type of cancers and inflammatory diseases are encompassed by the invention as long as they can be treated by the compositions of the invention.

### Pharmaceutical compositions and methods of therapy

The term "effective amount" means an amount when administered to the subject which results in beneficial or desired results, including clinical results, e.g., reduces the likelihood of developing the cancer or inhibits, suppresses or reduces the cancer (e.g., as determined by clinical symptoms or the amount of cancer cells) in a subject as compared to a control. Specifically, "treating a subject with a cancer" includes achieving, partially or substantially, one or more of the following: arresting the growth or spread of a cancer, reducing the extent of a cancer (e.g., reducing size of a tumor or reducing the number of affected sites), inhibiting the growth rate of a cancer, and ameliorating or improving a clinical symptom or indicator associated with a cancer (such as tissue or serum components). It also reduces the likelihood of reoccurrence of the cancer.

Generally, an effective amount of a compound of the invention varies depending upon various factors, such as the given drug or compound, the pharmaceutical formulation, the route of administration, the type of disease or disorder, the identity of the subject or host being treated, and the like, but can nevertheless be routinely determined by one skilled in the art. An effective amount of a compound of the present invention may be readily determined by one of ordinary skill by routine methods known in the art.

In an embodiment, an effective amount of a compound of the invention ranges from about 0.01 to about 1000 mg/kg body weight, alternatively about 0.05 to about 500 mg/kg body weight, alternatively about 0.1 to about 100 mg/kg body weight, alternatively about 0.1 to about 15 mg/kg body weight, alternatively about 1 to about 5 mg/kg body weight, and in another alternative, from about 2 to about 3 mg/kg body weight. The skilled artisan will appreciate that certain factors may influence the dosage required to effectively treat a subject suffering from cancer and these factors include, but are not limited to, the severity of the disease or disorder, previous treatments, the general health and/or age of the subject and other diseases present. Moreover, a "treatment" regime of a subject with an effective amount of the compound of the present invention may consist of a single administration, or alternatively comprise a series of applications. For example, the compound of the present invention may be administered at least once a week. However, in another embodiment, the compound may be administered to the subject from about one time per week to once daily for a given treatment. The length of the treatment period depends on a variety of factors, such as the severity of the disease, the age of the patient, the concentration and the activity of the compounds of the present invention, or a combination thereof. It will also be appreciated that the effective dosage of the compound used for the treatment or prophylaxis may increase or decrease over the course of a particular treatment or prophylaxis regime. Changes in dosage may result and become apparent by standard diagnostic assays known in the art. In some instances, chronic administration may be required.
As used herein, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, reducing the likelihood of the spread of the disease, delay or slowing of disease progression, amelioration or palliation of the disease state, and remission (whether partial or total), whether detectable or undetectable. "Treatment" can also mean prolonging survival as compared to expected survival if not receiving treatment. "Treatment" also includes reducing the likelihood of developing the disease or reducing the likelihood of reoccurrence of the disease.

The subjects to be treated are mammals, particularly humans in need of said treatment.

In one embodiment, the present invention is a mono-therapy where the pharmaceutical compositions of the invention are administered alone. Accordingly, in this embodiment, the compound of the invention is the only pharmaceutically active ingredient in the pharmaceutical compositions or the only pharmaceutically active ingredient administered to the subject.

In another embodiment, the method of the invention is a combinationtherapy with one or more of other therapeutically active drugs or therapies known in the art for treating the desired diseases or indications. In one example, one or more other anti-proliferative or anticancer therapies are combined with the compounds of the invention. In another example, the compounds disclosed herein are co-administered with one or more of other anticancer drugs known in the art. Anticancer therapies that may be used in combination with the compound of the invention include surgery, radiotherapy (including, but not limited to, gamma-radiation, neutron beam radiotherapy, electron beam radiotherapy, proton therapy, brachytherapy, and systemic radioactive isotopes) and endocrine therapy. Anticancer agents that may be used in combination with the compounds of the invention include biologic response modifiers (including, but not limited to, interferons, interleukins, and tumor necrosis factor (TNF)), hyperthermia and cryotherapy, agents to attenuate any adverse effects (e.g., antiemetics), and other approved chemotherapeutic drugs (e.g. taxol, 5-FU, and analogs thereof).

When the compounds of the invention are combined with other anticancer drugs, they can be administered contemperaneously. As used herein, "administered contemporaneously" means that two substances are administered to a subject such that they are both biologically active in the subject at the same time. The exact details of the administration will depend on the pharmacokinetics of the two substances in the presence of each other, and can include administering one substance within a period of time of one another, e.g., 24 hours of administration of the other, if the pharmacokinetics is suitable. Designs of suitable dosing regimens are routine for one skilled in the art. In particular embodiments, two substances will be administered substantially simultaneously, i.e. within minutes of each other, or in a single composition that comprises both substances. Alternatively, the two agents can be administered separately, such that only one is biologically active in the subject at the same time.

The compounds of the invention can be administered to a patient in a variety of forms depending on the selected route of administration, as will be understood by those skilled in the art. The compounds of the invention may be administered, for example, by oral, parenteral, buccal, sublingual, nasal, rectal, patch, pump or transdermal administration and the pharmaceutical compositions formulated accordingly. Parenteral administration includes intravenous, intraperitoneal, subcutaneous, intramuscular, transepithelial, nasal, intrapulmonary, intrathecal, rectal and topical modes of administration. Parenteral administration can be by continuous infusion over a selected period of time.

The compounds of the invention can be suitably formulated into pharmaceutical compositions for administration to a subject. The pharmaceutical compositions of the invention optionally include one or more pharmaceutically acceptable carriers and/or diluents therefor, such as lactose, starch, cellulose and dextrose. Other excipients, such as flavoring agents; sweeteners; and preservatives, such as methyl, ethyl, propyl and butyl parabens, can also be included. More complete listings of suitable excipients can be found in the Handbook of Pharmaceutical Excipients (5th Ed., Pharmaceutical Press (2005)). A person skilled in the art would know how to prepare formulations suitable for various types of administration routes. Conventional procedures and ingredients for the selection and preparation of suitable formulations are described, for example, in Remington's Pharmaceutical Sciences (2003 - 20th edition) and in The United States Pharmacopeia: The National Formulary (USP 24 NF 19) published in 1999. The carriers, diluents and/or excipients are "acceptable" in the sense of being compatible with the other ingredients of the pharmaceutical composition and not deleterious to the recipient thereof. Typically, for oral therapeutic administration, a compound of the invention may be incorporated with excipient and used in the form of ingestible tablets, buccal tablets, troches, capsules, elixirs, suspensions, syrups, wafers, and the like. Typically for parenteral administration, solutions of a compound of the invention can generally be prepared in water suitably mixed with a surfactant such as hydroxypropylcellulose. Dispersions can also be prepared in glycerol, liquid polyethylene glycols, DMSO and mixtures thereof with or without alcohol, and in oils. Under ordinary conditions of storage and use, these preparations contain a preservative to prevent the growth of microorganisms.

Typically, for injectable use, sterile aqueous solutions or dispersions of, and sterile powders of, a composition of the invention for the preparation of sterile injectable solutions or dispersions are formulated. For nasal administration, the compounds of the invention can be formulated as aerosols, drops, gels and powders. Aerosol formulations typically comprise a solution or fine suspension of the active substance in a physiologically acceptable aqueous or nonaqueous solvent and are usually presented in single or multidose quantities in sterile form in a sealed container, which can take the form of a cartridge or refill for use with an atomizing device. Alternatively, the sealed container may be a unitary dispensing device such as a single dose nasal inhaler or an aerosol dispenser fitted with a metering valve which is intended for disposal after use. Where the dosage form comprises an aerosol dispenser, it will contain a propellant which can be a compressed gas such as compressed air or an organic propellant such as fluorochlorohydrocarbon. The aerosol dosage forms can also take the form of a pump-atomizer. For buccal or sublingual administration, the compounds of the invention can be formulated with a carrier such as sugar, acacia, tragacanth, or gelatin and glycerine, as tablets, lozenges or pastilles.

For rectal administration, the compounds of the invention can be formulated in the form of suppositories containing a conventional suppository base such as cocoa butter.

The compounds of the invention can be formulated alone or for contemporaneous administration with other agents for treating cancer. Therefore, in another aspect, a pharmaceutical composition of the invention comprises a pharmaceutically acceptable carrier or diluent, a compound disclosed herein or a pharmaceutically acceptable salt thereof and another anti-cancer agent, for example, but not limited to a glucose metabolism inhibitor or taxol.

The invention described herein may suitably be practiced in the absence of any element or elements, limitation or limitations, not specifically disclosed herein. Thus, for example, the terms "comprising", "including", "containing", etc. shall be read-expansively-and-without terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions herein disclosed may be resorted to by those skilled in the art, and that such modifications and variations are considered to be within the scope of this invention.

### Predictive Medicine

The present invention also pertains to the field of predictive medicine in which diagnostic assays, prognostic assays are used for prognostic (predictive) purposes to thereby treat an individual prophylactically or to decide whether a treatment is of value at all due to a supposed ineffectiveness of a treatment. Accordingly, one aspect of the present invention relates to diagnostic assays for determining expression of a polypeptide or nucleic acid of the invention in the context of a biological sample (e.g., blood, serum, cells, tissue) to thereby determine whether an individual is afflicted with a disease or disorder, or is at risk of developing a disorder, associated with aberrant expression of DAPK, such as a proliferative disorder, e.g., cancer. The invention also provides for prognostic (or predictive) assays for determining whether an individual is at risk of developing a disorder associated with aberrant expression of DAPK. Such assays can be used for prognostic or predictive purpose to thereby prophylactically treat an individual prior to the onset of a disorder characterized by or associated with aberrant expression or activity of a polypeptide of the invention.

Yet another aspect of the invention pertains to a method for determining the overall survival time of a subject suffering from cancer in order to decide whether a treatment is of value at all due to a supposed ineffectiveness of a treatment or an unexpected short survival time

### Prognostic Assays

The prognostic assays described herein, for example, can be used to identify a subject having or at risk of developing disorders such as disorders discussed herein.

In another example, prognostic assays described herein can be used to identify a subject having or at risk of developing related disorders associated with aberrant expression of DAPK.

In another example, prognostic assays described herein can be used for determining the overall survival time of a subject having or at risk of developing disorders associated with aberrant expression of DAPK.

The term "prognosis", or grammatical variants thereof, as used herein refers to a prediction of the probable course and outcome of a clinical condition or disease. A prognosis of a patient is usually made by evaluating factors or symptoms of a disease that are indicative of a favorable or unfavorable course or outcome of the disease. The term "prognosis" does not refer to the ability to predict the course or outcome of a condition with 100% accuracy. Instead, the term "prognosis" refers to an increased probability that a certain course or outcome will occur; that is, that a course or outcome is more likely to occur in a patient exhibiting a given condition, when compared to those individuals not exhibiting the condition.

Furthermore, the prognostic assays described herein can be used for designing a personalized therapy for a subject suffering from cancer to determine whether a subject can be administered an agent (e.g., an agonist, antagonist, peptidomimetic, protein, peptide, nucleic acid, small molecule, or other drug candidate) to treat a disease or disorder associated with aberrant expression of DAPK. For example, such methods can be used to determine whether a subject can be effectively treated with a specific agent or class of agents (e.g., agents of a type which decrease activity of the polypeptide). Thus, the present invention provides methods for determining whether a subject can be effectively treated with an agent for a disorder associated with aberrant expression or activity of a polypeptide of the invention in which a test sample is obtained and the polypeptide or nucleic acid encoding the polypeptide is detected (e.g., wherein the presence of the polypeptide or nucleic acid is diagnostic for a subject that can be administered the agent to treat a disorder associated with aberrant expression or activity of the polypeptide).

An exemplary method for detecting the presence or absence of a polypeptide or nucleic acid of the invention in a biological sample involves obtaining a biological sample from a test subject and contacting the biological sample with a compound or an agent capable of detecting a DAPK polypeptide or nucleic acid (e.g., mRNA, genomic DNA) such that the presence of said polypeptide or nucleic acid of the invention is detected and quantified in the biological sample. A preferred agent for detecting and quantifying mRNA or genomic DNA encoding DAPK is a labeled nucleic acid probe capable of hybridizing to mRNA or genomic DNA encoding DAPK. The nucleic acid probe can be, for example, a full-length cDNA or a portion thereof, such as an oligonucleotide of at least 15, 30, 50, 100, 250 or 500 contiguous nucleotides in length and sufficient to specifically hybridize under stringent conditions to a mRNA or genomic DNA encoding DAPK. Other suitable probes for use in the diagnostic assays of the invention are known to the skilled expert.

A preferred agent for detecting DAPK is an antibody capable of binding DAPK, preferably an antibody with a detectable label. Antibodies can be polyclonal, or more preferably, monoclonal. An intact antibody, or a fragment thereof (e.g., Fab or F(ab')2) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject associated with or supposed to be associated with cancer. That is, the predictive methods of the invention can be used to detect mRNA, protein, or genomic DNA in a biological sample in vitro as well as in vivo. For example, in vitro techniques for detection of mRNA include Northern hybridizations and in situ hybridizations. In vitro techniques for detection of a polypeptide of the invention include enzyme linked immunosorbent assays (ELISAs), Western blots, immunoprecipitations and immunofluorescence. In vitro techniques for detection of genomic DNA include Southern hybridizations. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a tissue sample isolated by conventional means from a subject believed to be associated with a tumor.

In another embodiment, the invention further involves obtaining a control biological sample from a control subject, contacting the control sample with a compound or agent capable of detecting a polypeptide of the invention or mRNA or genomic DNA encoding a polypeptide of the invention, such that the presence of the polypeptide or mRNA or genomic DNA encoding the polypeptide is detected in the biological sample, and comparing the presence of the polypeptide or mRNA or genomic DNA encoding the polypeptide in the control sample with the presence of the polypeptide or mRNA or genomic DNA encoding the polypeptide in the test sample.

### Kits

The invention also encompasses kits for detecting the presence of a polypeptide or nucleic acid of the invention in a biological sample (a test sample). Such kits can be used to determine if a subject is suffering from or is at increased risk of developing a cancer associated with aberrant expression of DAPK of the invention.

For example, kits can be used to determine if a subject is suffering from or is at increased risk of developing a cancer associated with aberrant expression of DAPK of the invention which is associated with aberrant expression of developing a cancer associated with aberrant expression of DAPK of the invention.

In another example, kits can be used to determine the overall survival time of a subject suffering from cancer which is associated with aberrant expression of DAPK. In another example, kits can be used for designing a personalized therapy for a subject suffering from cancer which is associated with aberrant expression of DAPK.

The kit, for example, can comprise a labeled compound or agent capable of detecting the polypeptide or mRNA encoding the polypeptide in a biological sample and means for determining the amount of the polypeptide or mRNA in the sample (e.g., an antibody which binds the polypeptide or an oligonucleotide probe which binds to DNA or mRNA encoding the polypeptide). Kits can also include instructions for observing that the tested subject is suffering from or is at risk of developing a disorder associated with aberrant expression of the polypeptide if the amount of the polypeptide or mRNA encoding the polypeptide is above or below a normal level.

For antibody-based kits, the kit can comprise, for example: (1) a first antibody (e.g., attached to a solid support) which binds to DAPK; and, optionally, (2) a second, different antibody which binds to either the polypeptide or the first antibody and is conjugated to a detectable agent.

For oligonucleotide-based kits, the kit can comprise, for example: (1) an oligonucleotide, e.g., a detectably labeled oligonucleotide, which hybridizes to a nucleic acid sequence encoding DAPK or (2) a pair of primers useful for amplifying a nucleic acid molecule encoding DAPK. The kit can also comprise, e.g., a buffering agent, a preservative, or a protein stabilizing agent. The kit can also comprise components necessary for detecting the detectable agent (e.g., an enzyme or a substrate). The kit can also contain a control sample or a series of control samples which can be assayed and compared to the test sample contained. Each component of the kit is usually enclosed within an individual container and all of the various containers are within a single package along with instructions for observing whether the tested subject is suffering from or is at risk of developing a disorder associated with aberrant expression of DAPK.

The methods described herein may be performed, for example, by utilizing pre-packaged kits comprising at least one probe nucleic acid or antibody reagent described herein, which may be conveniently used, e.g., in clinical settings to diagnose patients exhibiting symptoms or family history of a disease or illness involving an aberrant expression of DAPK. Furthermore, any cell type or tissue, in which DAPK is expressed, may be utilized in the prognostic assays described herein.

In order to provide a full disclosure of the invention, the following additional information is given.

Tumor necrosis factor-α (TNF) is a pleiotropic cytokine that participates in several biological functions, including inflammation, apoptosis, growth and differentiation.^{1,2} It activates the inflammatory pathway via nuclear factor-κB (NFκB) or apoptosis via caspases, which depends on the particular proteins recruited to the receptors.^{3,4} Moreover, TNF has been implicated in the pathogenesis of various inflammatory diseases like ulcerative colitis (UC), Crohn's disease, and rheumatoid arthritis.^{5,6}
The etiology of UC still remains obscure; however, genetic, immunologic, and environmental factors probably contribute to disease pathogenesis.⁷ An imbalance between pro- and anti-inflammatory cytokines and a defect in intestinal barrier function cause chronic recurrent inflammation of the gut.^{8,9} As inflammation compromises gut homeostasis and is also associated with cancer progression,¹⁰ it is important to understand the role of key molecules that are involved in the activation of the inflammatory cascade.
The Death associated protein kinase (DAPK) is a calcium/calmodulin-regulated serine/threonine kinase with a protective role during chronic inflammation in UC and UCC (UC-associated carcinoma).¹¹ Interestingly, DAPK can regulate inflammation either positively through NLRP3 inflammasome formation¹² or negatively through inhibition of NFκB.^{13,14} TNF activates NFκB by phosphorylating the inhibitor of NFκB (IκBα), which is then degraded in an ubiquitin-mediated step. Activated NFκB initiates the transcription of target genes including the pro-inflammatory cytokine IL-6.^{1,15,16} IL-6 is shown to be a major mediator of inflammation through the activation of the signal transducer and activator of transcription 3 (STAT3) pathway.¹⁷⁻¹⁹ Subsequent to the cytokine action, Janus kinases (JAK) phosphorylate and activate STAT3 at Y705.^{19,20} The activation of STAT3 leads to its dimerization, followed by nuclear translocation and DNA binding to regulate target gene expression.²¹ Until now only a few negative regulators of STAT3 activity have been reported such as SOCS₃, PIAS, ERK, KAPI, and protein phosphatases.²² The TNF→NFκB and IL-STAT3 pathways are shown to play a crucial role in promoting colitis-associated carcinoma formation.²³⁻²⁹
Until now, studies related to the pathogenesis of inflammatory bowel disease (IBD) were carried out using either immune or cancer cells or mouse models, while non-immune cells, including epithelial cells, are considered to play a rather passive role.³⁰ However, accumulating evidence suggests that IEC are more than just a barrier and seem to be equally competent in IBD pathogenesis.³⁰ Therefore, we studied TNF-induced signaling in normal human colon epithelial cells (HCEC) and proved its in vivo relevance in UC tissues.
Our results demonstrate that DAPK and pSTAT3^{γ705} were activated under inflammation both in vitro and in *vivo.* We also report a novel negative regulation principle between DAPK and STAT3, which might balance TNF-induced inflammation. The divergent expression pattern of these proteins in UC and UCC emphasizes their important role in the inflammation-associated transformation process.

### MATERIALS AND METHODS

### Material

General cell culture reagents like PBS, Trypsin, and Basal HCEC medium were obtained from PAN (PAN Biotech GmbH, Aidenbach, Germany). Other medium supplements of HCEC medium were obtained from Sigma (Saint Louis, Missouri, USA). Human TNF (Immuno Tools GmbH, Friesoythe, Germany), Human IL-6, Human IL-6 mAB (R&D Systems, Minneapolis, MN, USA), DAPK inhibitor - (4Z)-2-phenyl-4-(pyridine-3-ylmethylidene)-4,5-dihydro-1,3-oxazol-5-one (MolPort), JAK inhibitor - Tyrphostin AG 490 (Sigma), Stattic (Calbiochem, Darmstadt, Germany) were obtained from the sources mentioned.

### Cell culture

HCEC cells were kindly provided by Professor Steinberg Pablo (Institute for Food Toxicology and Analytical Chemistry, University of Veterinary Medicine Hannover, Germany) and maintained as previously described.³¹ After 24 hours of seeding, cells were either stimulated with 0.66 ng/ml TNF (ImmunoTools) for various time points. For inhibitor experiments cells were pre-incubated for one to two hours with the corresponding inhibitors.

### Patient samples

Gut specimens were obtained from UC or non-IBD control patients and analyzed by immunohistochemistry. The UC group included 140 samples from 120 patients (average age: 51±32) with inactive UC (n=49), low-active UC (n=41), highly-active UC (n=15), dysplasia-associated lesion or mass (DALM; n=11) and UCC (n=24). The control group consisted of patients that underwent control colonoscopy for cancer prevention (n=11; average age: 64±21 years). IEC preparations from gut specimens of UC patients (n=4) were assessed by Western blotting. Details like age, gender, and histological/pathological activity are given in the Supplemental material (see Supplemental Table S1 at http://ajp.amjpathol.org). The Disease activity score was calculated as previously described³² for available cases. The present study was performed following approval by our local ethical committee.

### Immunohistochemistry (IHC) and Histological score

IHC was used to detect the expression of DAPK, pSTAT3^{γ705}, and TNF in the formalin-fixed, paraffin-embedded tissue microarrays (TMA). 2-4 µM thick sections were dewaxed at 72°C for 30 minutes and then incubated in fresh xylene 2X, 5 minutes. Tissue sections were rehydrated in descending concentrations of ethanol (96% to 70%). Antigen was retrieved by heating in a pressure cooker (1 mM Tris-EDTA buffer, 120°C, 5 min). Endogenous peroxidases and non-specific biding sites were blocked by incubating the slices with blocking solution (Dako). All slices were then incubated with primary antibodies anti-DAPK (1:100), pSTAT3^{γ705} (1:50), and anti-TNF (1:300) at RT for 30 minutes. After washing with washing buffer (Dako), sections were incubated with secondary antibody at RT for 30 minutes. Secondary antibodies were EnVision+System HRP linked (goat-anti-mouse or goat-anti-rabbit; Dako) and positive immunoreactivity was detected using DAB+ (Dako) or fast red (Dako) as chromogen substrate. Nuclei were counterstained with hematoxylin (Dako). Appropriate positive and negative controls were included in each run of IHC. Histological evaluation was performed by reviewing the hematoxylin-eosin (HE) stained tissue sections. The percentage of epithelial cells that stained positive (immunoreactivity above the background) was quantified/scored in a blinded manner (TTR, AA, AH).

### RNA isolation and real time RT-PCR

Expression of IL-6, IL-8, and DAPK mRNA were analyzed by real time RT-PCR. RNA isolation (mRNeasy RNA isolation kit) and cDNA synthesis (Quantitect reverse transcriptase kit) were performed according to the manufacturer's instructions (Qiagen, Hilden, Germany). 1 µl of cDNA was amplified in a thermal cycler (Biorad CFX-96) with corresponding primers in a total volume of 25 µl using Quantifast SYBR green kit (Qiagen) under the following conditions: 95°C for 5 minutes followed by 26-40 cycles of 95°C for 10 s and 60°C for 30 s. The following primers were used, forward and reverse respectively: DAPK, 5'-CCTTGCAAGACTTCGAAAGGATA-3' and 5'-GATCCCGAGTGGCCAAA-3'; IL-6, 5'-ATGAACTCCTTCTCCACAAGCGC-3' and 5'-CAGTCCAGCCTGAGGGCTCTTC-3'; IL-8, 5'-CCAAGGAAAACTGGGTGCAGAG-3' and 5'-ACAAGTCCTTGTTCCACTGTGCC-3'; ß2microglobulin (house-keeping gene), 5'-CCAGCAGAGAATGGAAAGTC-3' and 5'-GATGCTGCTTACATGTCTCG-3'; murine DAPK, 5'-TGCACAACAGCTACACAGCA-3' and 5'-GACCAGACGCTGGATGTCTT-3'; murine GAPDH (house-keeping gene), 5'-TGTGTCCGTCGTGGATCTGA -3' and 5'-CCTGCTTCACCACCTTCTTGA -3'. The results were expressed as fold induction compared to unstimulated cells after normalising to house-keeping gene. All primers were purchased from metabion (metabion international AG, Martinsried, Germany).

### Western Blotting

Protein concentration was measured in duplicates using Bio-rad Dc Protein Assay (BioRad Laboratories, Hercules, CA). Equal amounts of protein were separated by 10% or 12% SDS-PAGE using Laemmli buffer system. Proteins were transferred electrophoretically to nitrocellulose membrane (Millipore Corporation, Billerica, MA, USA) and detected as recently described³³ using following antibodies, anti-DAPK (BD Transduction Laboratories, Lexington NY), anti-pDAPK^{S308} (Sigma), anti-STAT3, anti-pSTAT3^{γ705}, anti-caspase3 (Cell Signaling Technology Inc.), anti-ß-actin (Sigma), or anti-GAPDH (Abnova GmbH, Germany).

### Enzyme-Linked Immunosorbent Assay (ELISA)

IL-6 and IL-8 secretion was analyzed by ELISA according to the manufacturer's instructions (BD Biosciences; Heidelberg, Germany). Briefly, flat-bottom 96-well microtiter plates (BD Biosciences, Heidelberg, Germany) were coated with 100 µl capture antibody (1:250 diluted in Na₂HCO₃) and incubated at 4°C for overnight. After blocking (300 µl 3% BSA in PBS, 2 hours, RT) and washing (0.1% Tween in PBS, (PBS-T), 100 µl of undiluted or diluted supernatant was added and incubated (2 hours, RT or overnight 4°C). Thereafter, wells were washed and incubated (1.5 hours, RT) with detection antibody (1:250 diluted in 1% BSA in PBS-T) + enzyme reagent (Streptavidin-horseradish peroxidase conjugate; 1:250 diluted in detection antibody). After washing, TMB (1:1) substrate was added to each well and incubated in a dark for 20-30 minutes. Reaction was stopped with stop solution (100 µl 1 M H₂SO₄) and absorption was measured using spectrophotometer (Victor X3, Perkin-elmer) at a wavelength of 450 nm with a wavelength correction at 570 nm.

### siRNA transfection

Silencing of DAPK expression in HCEC cells was performed by the **siRNA** technique according to the manufacturer's instructions (Dharmacon, Chicago, IL, USA). Briefly, the HCEC cells were grown to 60% confluence in a 6-well tissue culture plates. Transfection mixture was prepared in a final volume of 400 µl to achieve final **siRNA** concentration of 100 nM. This mixture was incubated for 30 minutes at RT to allow complex formation and then added onto the cells drop by drop. After 24 hours of incubation, medium was replenished and subsequently treated with 0.66 ng/ml TNF for 24 and 48 hours. A non-specific control **siRNA** SMARTpool (100 nM, Dharmacon) was used as a negative control. At the end of the incubation period supernatants and cells were harvested and stored at -80°C until analyzed further. The knockdown efficiency was determined by Western blotting.

### Immunoprecipitation (IP)

IP was performed using the Dynabeads Protein G magnetic separation kit according the manufacturer's instructions (Invitrogen, Karlsruhe, Germany). Briefly, protein G magnetic Dynabeads were coated with DAPK antibody (1:500 to that of protein concentration) for 2 hours with rotation at RT and Dynabeads-antibody complexes were washed. 600-900 µg of protein lysate was added to the Dynabeads-antibody complex and gently resuspend by pipetting. The Dynabeads-antibody-antigen complex was incubated overnight at 4°C with rotation. The Dynabeads-antibody-antigen complexes were washed and immunoprecipitates were eluted in 20 µl elution buffer. The proteins were separated by SDS-PAGE and Western blot analysis was performed using anti-STAT3 antibody.

### Structural analysis of DAPK-STAT3 complex

To understand the interactions between STAT3 and DAPK, the structures of JAK and STAT3 deposited in the PDB (JAK: 3EYG- Crystal structures of JAK1 and JAK2 inhibitor complexes at 1.9 A resolution; STAT3: 1BG1- X ray structure of Transcription Factor -STAT3B- DNA complex at 2.25 Å resolution) were considered. The JAK2 inhibitor was removed from the structure 3EYG and the DNA was removed from the structure 1BG1 to facilitate analysis of the JAK-STAT3 complex.
The catalytic domain of DAPK (PDB:1JKS-Xray structure of the catalytic domain of human DAPK at 1.5 Å resolution) was then docked to STAT3 monomer and ranking was done using ClusPro server. The STAT3-STAT3 dimer was then formed followed by the creation of a STAT3-DAPK-STAT3 complex through docking. The docking, energy filtering and ranking of the complexes of these structures were done by the ClusPro server.³⁴ In all cases, top 1000 structures were chosen after energy filtering (electrostatics), clustered and ranked according to cluster sizes. The hydrogen bond interactions in the STAT3-STAT3 dimer and in the STAT3-DAPK-STAT3 complex were analyzed using HBOND Calculator. The hydrophobic interactions between these complexes were analyzed using the PIC Server. All renderings were done using CHIMERA.³⁵

### Preparation of cytoplasmic and nuclear lysates

Cell pellets were resuspended in 300 µl of cold Buffer A (10 mM Tris pH 7.9; 10 mM KCl; 1.5 mM MgCl2; 10% Glycerol; 10 mM K₂HPO₄; 1 mM Na₃VO₄; 10 mM NaF; 0.5 mM DTT; 1 mM ABSF; 1X-protease inhibitors) with 0.125% NP-40 and incubated on ice for 5 minutes. The homogenate was centrifuged for 10 minutes at 1000g at 4°C and the supernatant containing cytoplasmic proteins was collected into a fresh tube. The nuclear pellet was washed once with Buffer A and then resuspended in 50-100 µl of Buffer C (20 mM Tris pH 7.9; 0.42 mM NaCl; 1.5 mM MgCl₂; 2 1 mM EDTA; 10% Glycerol; 10 mM K₂HPO₄; 1 mM Na₃VO₄; 10 mM NaF; 0.5 mM DTT; 1 mM ABSF; 1X-protease inhibitors) and sonicated. The nuclear extract is centrifuged for 10 minutes at 12000g at 4°C and the supernatant with nuclear proteins is transferred into fresh tube.

### Electrophoretic Mobility Shift Assay (EMSA)

STAT3 DNA binding activity was evaluated using non-radioactive EMSA, performed as recently described.³⁶ For performing EMSA 10 µg of nuclear protein was incubated with IRDye 700-labelled double stranded STAT3 consensus or mutant oligonucleotides (0-5 µl of 50 nM) in 20 µl of incubation buffer containing 2 µl binding buffer (100 mM Tris; 500 mM NaCl; 100 µM EDTA; 10 mM DTT; 50% Glycerol), 1 µl of 1% NP-40, 1 µl of 2.5% Tween, 1 µl of poly dI-dC (2 µg/ml), 2 µl BSA (10 mg/ml), 1 µl of 2.5% Tween 20, and 1 µl of 1% NP-40. The sequence of the probes used was as follows, consensus sense 5'-GATCCTTCTGGGAATTCCTAGATC-3'; and mutant sense 5'-GATCCTTCTGGGCCGTCCTAGATC-3'. After 30 minutes of incubation at 18°C, 300 rpm, samples were loaded and run on a 4% Lipage gel at 150 V, 4°C for 2hours. DNA-protein complexes were detected using Odyssey system (Licor, Biosciences GmbH, Bad Homburg, Germany). The specificity of the complexes was also verified by competition experiments, co-incubating unlabelled consensus (100x) with labeled consensus oligos.

### Chromatin Immunoprecipitation (ChIP)

CHIP experiments were performed as previously described³⁷ using the ChIP-IT express kit (Active Motif, Rixensart, Belgium). At the end of the incubation period, cells were treated with 1% formaldehyde for 10 minutes at RT to cross-link DNA and associated proteins. Chromatin was extracted and sonicated on ice with 6x, 15sec, 30% power, 01 01 pulse to using HTU Soni 130 (G. Heinemann) sonicator to obtain DNA fragments of average size of 500 bp. Immunoprecipitations were performed by incubating 60 µl chromatin and 25 µl protein G magnetic beads with 15 µl pSTAT3^{γ705} antibody (Cell Signaling) or negative control IgG of equivalent concentration overnight at 4°C on a rotating platform. The beads were washed, protein-DNA cross-links were reversed and 5 µl DNA from the input and IP samples were subjected to real-time or end-point PCR using primers corresponding to two different regions of the human DAPK promoter. The following primers were used region 1 (-1821/-1472) forward primer, 5'-TGCAGTGAGCCAAGATTTCA-3' and reverse primer, 5'-TTCCGATCCATACCGTTGTT-3' and region 2 (-631/-351) forward primer, 5'-ATGAGGTACGCTCCCTTCCT-3' and reverse primer, 5'-TCGTCCCGAGATGTGTACTG-3'. PCR products were analyzed by agarose gel electrophoresis in case of end-point PCR and in case of real time PCR data were expressed as fold increase over unstimulated cells. All ChIP assays were performed four times.

### Experimental mouse models and IEC isolation

Mice carrying a loxP flanked Stat3 allele (Stat3 wt) were kindly provided by S. Akira.³⁸ C57BL/6 mice carrying the sequence for the enzyme cre-recombinase under control of the Villin promoter (Villin-Cre mice) were described earlier.³⁹ Stat3 wt mice were crossbred with Villin-Cre mice. In this way, conditional knockout mice with IEC-specific deletion of Stat3 activity (Stat3^{IEC-KO}) were generated. We have previously shown,⁴⁰ that normal STAT3^{IEC-KO} mice do not develop spontaneous colitis. Histology indicates that there is no underlying inflammation present in unchallenged mice. All mice were kept in individually ventilated cages in compliance with the Animal Welfare Act.

### Isolation of intestinal epithelial cells:

Intestinal epithelial cells were isolated by carefully removing the entire intestine from the mouse corpse. The intestine was inverted and washed free of stool in phosphate buffered saline. Intercellular connections were destroyed by incubating the inverted gut tissue in pre-warmed isolation solution (HBSS (PAA), 1mm EGTA (Sigma), 2mM EDTA (Sigma) and 10 % FCS (PAA)) and shaking at 200 rpm for 10 minutes at 37°C. Subsequently, the isolated cells were pelleted at 1,200 rpm and 4°C for 5 minutes and washed twice with phosphate buffered saline followed by centrifugation

### Experimental model of intestinal inflammation:

To induce experimental colitis, mice were treated with dextran sodium sulphate (DSS, MP Biomedicals). 2-3 % DSS were dissolved in sterile drinking water and the solution was given to the mice in drinking water bottles for 7 days and renewed every second day. Mouse body weight was monitored regularly to determine the state of health of the mice. Development of colitis was followed by regular colonoscopy and the severity of colitis in live mice was scored as previously described.^{41,42} DSS administration at the specified conditions caused moderate inflammation, and the weight loss per mouse was less than 10 percent.

### Experimental model of colon carcinogenesis:

Experimental colitis-associated tumorigenesis was performed as previously described.⁴³ In brief, 10mg/kg Azoxymethane (AOM; Sigma) was injected intraperitoneally into 6-8 week old C57BL/6J mice, followed by 3 cycles of DSS in drinking water. Each DSS-cycle was composed of DSS (2.5 % (w/v); MP Biomedicals) in drinking water for 7 days, followed by a recovery phase with regular drinking water for 14 days. All tumors were harvested at day 65-70.

### IEC isolation from UC patients

Intestinal tissue was obtained from patients with inflammatory bowel diseases who had to undergo surgery for various reasons (e.g. stenosis, fistulae, perforation, and therapy refractory disease). The gut specimen was initially thoroughly washed with sterile PBS and the mesenteric fat tissue was carefully removed. The intestinal mucosal layer was opened longitudinally and removed from the underlying muscular layer and thereafter cut into appx. 1 cm x 4 cm stripes. After incubation with 20 ml PBS and 31 mg dithiothreitol (DTT) for 30 minutes at 37°C at 200 rpm, the mucosa was again washed with PBS. Next the mucosal stripes were incubated in 20 ml PBS with 80 µl 0,5 M EDTA (2 mM) for 15 minutes at 37°C with 200 rpm. Afterwards colonic epithelial crypts were collected from this suspension and the washing steps with EDTA were repeated till the resulting suspension appeared clear of the isolated epithelial cells.
Isolated colonic epithelial crypts and/or cells were pelleted and resuspended in 10-25 ml of DMEM medium and further enriched using density gradient centrifugation. 3ml of cell suspension was overlaid on the top of the Percoll of 1.077 gm/ml density and centrifuged at 2500 rpm for 20 minutes at room temperature. Cells bands at density level 1.077 gm/ml were collected cautiously and washed with PBS. A small fraction of cell suspension was spread on a microscopic glass slides by centrifugation via cytospin at 1000 rpm for 10 minutes and stained with pan-cytokeratin, cytokeratin-19, and CD34 antibodies. Another small fraction of cell suspension was stained with EpCAM (CD326)-FITC and assessed by flow cytometry. Remaining cell suspension was pelleted and used for protein extraction.

### Apoptosis and Cell viability assay

Experimental procedures have previously been described.⁴⁴ Apoptosis was measured using Annexin-V-FLUOS kit or M30 cytodeath detection kit (Roche Diagnostic GmbH, Penzberg, Germany). At the end of the treatment, cells were stained with 100 µl annexin V/PI solution (20 µl FITC-conjugated annexin V reagent (20 µg/ml) + 20 µl PI reagent (50 µg/ml in 1 ml of dilution/HEPES buffer) for 15 minutes at RT in dark. In case of M30 staining cells were fixed with ice-cold methanol for 30 minutes at -20°C. After washing, the cells were incubated with M30 cytodeath antibody working solution [1:250 in incubation buffer (PBS+1% BSA+0.1% tween)] for 30 minutes at RT. In both cases, the cell suspension was diluted by adding appropriate amount of dilution buffer and analyzed using FACS Calibur flow cytometer (BD, CA).
Cell viability was assessed by crystal violet staining. At the end of the incubation period supernatants were discarded, cells were washed twice with pre-warmed PBS and stained with a crystal violet solution (0.5% crystal violet in 20% methanol) for 15 minutes. After removal of crystal violet solution, the plates were washed with tap water and air dried. The dye was eluted with methanol for 15 minutes and absorbance was measured at 595 nm using a microtiter plate reader (Victor X₃, Perkin-elmer)

### Statistical analysis

Statistical analysis was performed using SPSS (SPSS Inc., Chicago, IL, USA). The Student's t-test or the Mann-Whitney U-test was used for single comparisons and analysis of variance (ANOVA) followed by Tukey's HSD, Dunnett's t, and Student-Newman-Keuls post hoc tests were used for multiple comparisons. p Values ≤ 0.05 were considered statistically significant. Scatter-plots and Mann-Whitney tests were done by using GraphPad Prism 7.1.

### RESULTS

### Expression of DAPK and pSTAT3^{Y705} is augmented in IEC of UC and UCC

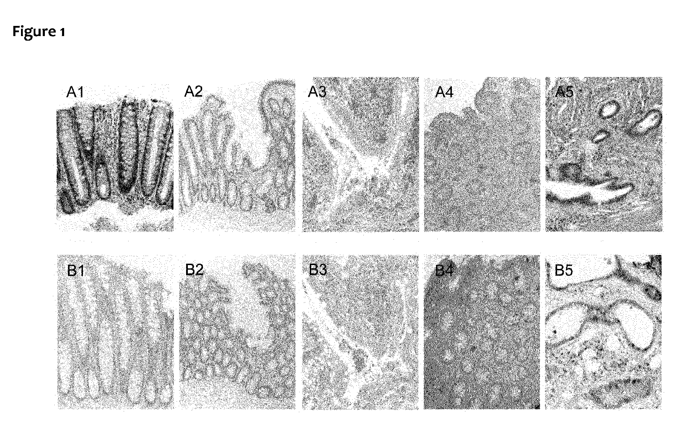

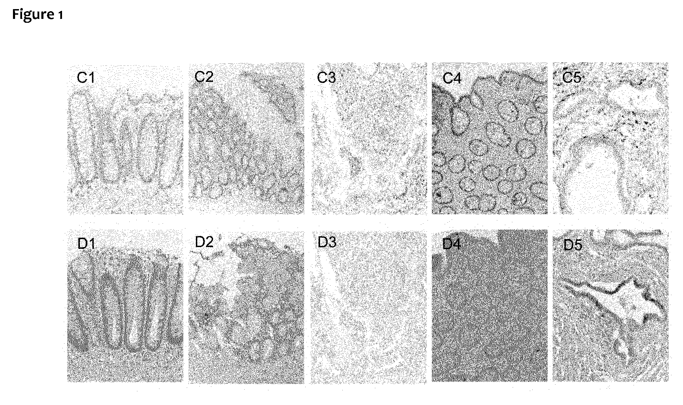

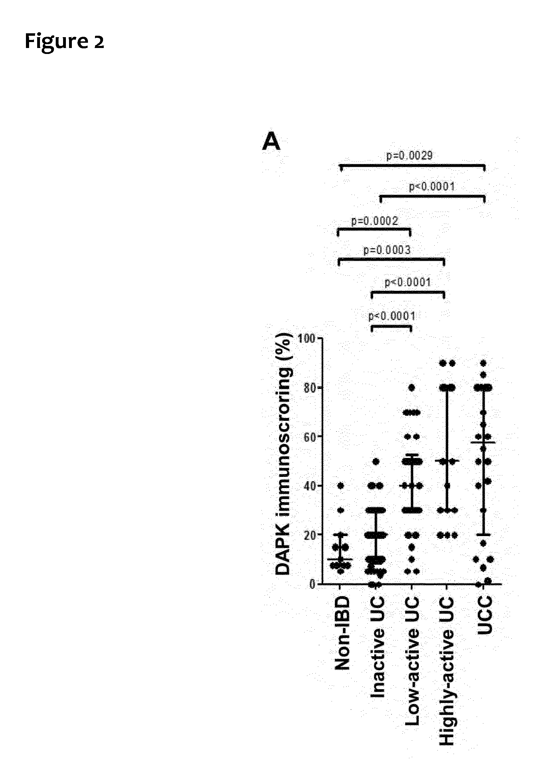

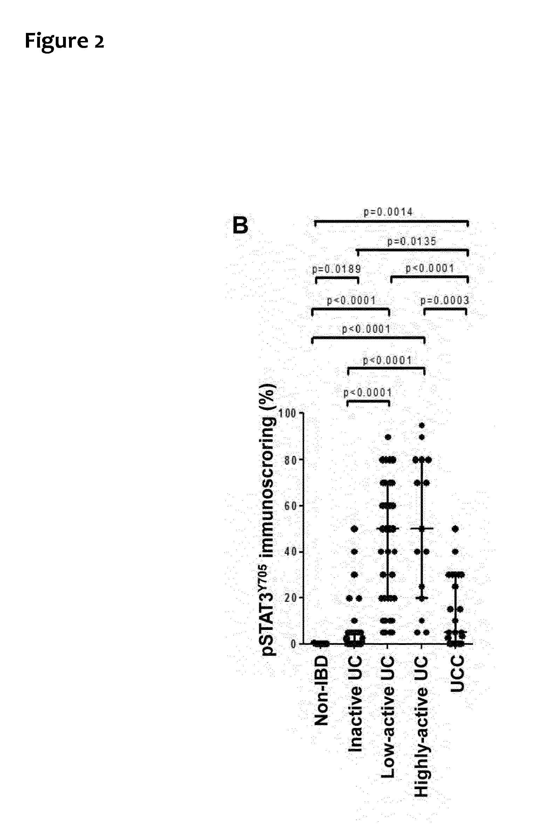

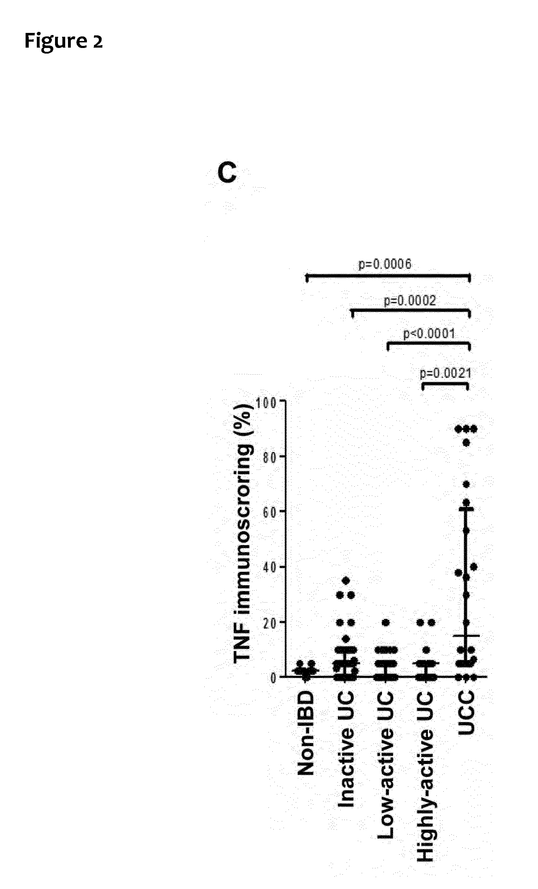

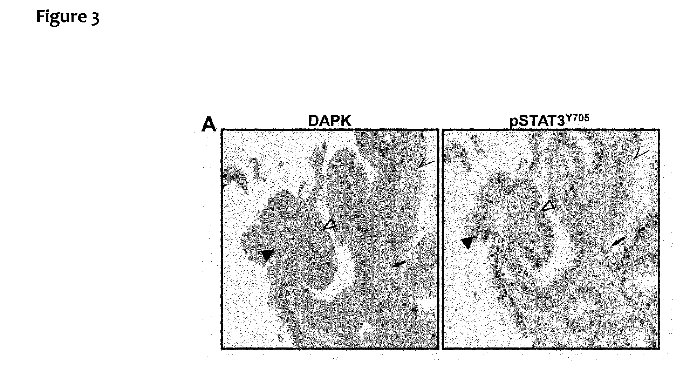

We have previously shown an increase in DAPK expression in UC-associated tumors¹¹ and STAT3 activation in a colitis mouse model.⁴⁰ To better understand the role of these two proteins in the inflammation-associated process we evaluated their immunohistochemical expression in IEC of human gut specimens from non-IBD, inactive UC, low-active UC, highly-active UC and UCC patients. HE staining depicts the inflammation grade of the sections (Figure 1A-E). Up to 80% of the IEC in the active UC and UCC specimens expressed DAPK in the cytoplasm, a percentage that was significantly higher than that (less than 20%) in non-IBD/inactive UC samples (Figure 1F-J,2A). As in the case of DAPK, a strong pSTAT3^{Y705} expression was observed in up to 80% of IEC present in the active UC samples, while less than 3% of IEC were positive in non-IBD/inactive UC specimens. However, in contrast to DAPK, most of the carcinoma specimens lost pSTAT3^{Y705} expression and only up to 20% of the IEC in the samples were scored positive, which was significantly lower if compared to the percentage observed in active UC specimens, but still significantly higher when compared to that of non-IBD/inactive UC samples (Figure 1K-O,2B). The increase of epithelial TNF expression was substantial and significant only in UCC when compared to the non-IBD/inactive UC/active UC specimens (Figure 1P-T,2C). The expression pattern of all the three markers (DAPK, pSTAT3^{Y705}, and TNF) did not differ from low-active UC to highly-active UC.
Although DAPK as well as pSTAT3⁷⁰⁵ protein expression increased with the severity of inflammation, this common expression pattern seemed to be lost between UC and UCC. To clarify this issue, we analyzed the expression of DAPK and pSTAT3^{Y705} in DALM samples, which represent UC-associated intraepithelial neoplasia. Indeed a heterogeneous pattern of staining was observed in some of the DALM samples, thereby showing all possible combinatory patterns for both proteins (Figure 3A).
In a next step, human IEC were isolated from gut specimen of UC patients from macroscopically inflamed and non-inflamed colonic mucosa. We observed an inverse correlation between pSTAT3^{Y705} and DAPK: There were two UC patient samples showing enhanced STAT3 phosphorylation but diminished DAPK expression in IEC from the inflammatory region compared to normal mucosa. Vice versa, in two UCC patients (patients 3&4), STAT3^{Y705} phosphorylation appeared to be diminished but DAPK expression was up-regulated in IEC from inflamed. In accordance with the observation that the inactive pDAPK^{S308} form was almost completely lost in the course of inflammation, the DAPK level increased and kinase activity was enhanced in inflamed IEC. Obviously, DAPK resumes the control during the malignant transformation process and pSTAT3^{Y705} activation seems to be no longer necessary for tumor survival.
We further investigated the influence of STAT3 on DAPK expression in an in *vivo* mouse model. IEC were isolated from wildtype (wt) and STAT3^{IEC-KO} mice. DAPK expression was significantly higher both at the mRNA (1-4-fold) and protein (2.4-fold) level in STAT3^{IEC-KO} mice than in wt mice, suggesting that DAPK expression might be negatively regulated by STAT3.

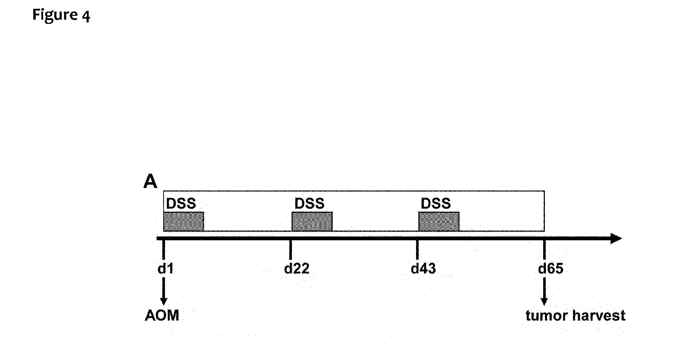

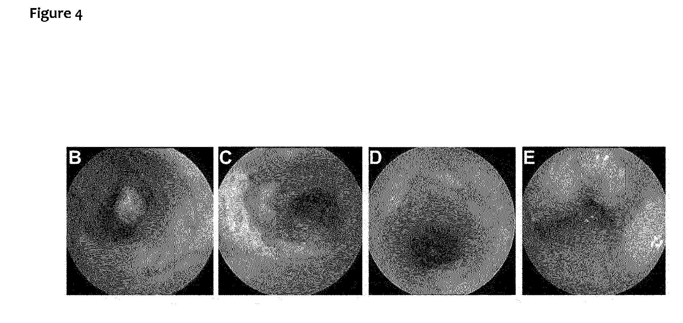

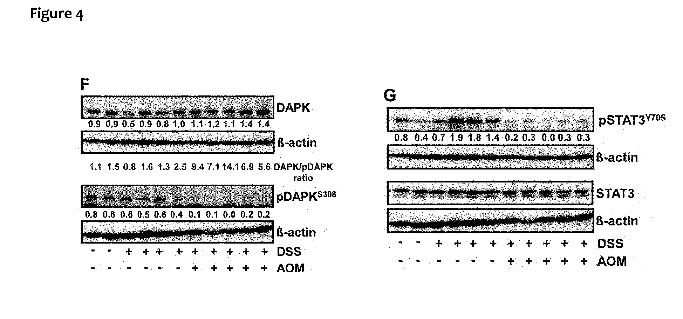

Expression/activation of DAPK/STAT3 is modulated during colitis-associated carcinogenesis. Tissue extracts from colon of control and DSS mice as well as AOM+DSS tumors were assessed by Western blotting to evaluate the modulation of DAPK/STAT3 expression/activation following the transformation from inflammation to cancer. Endoscopy images demonstrate the induction of inflammation and tumor formation by treatment with AOM+DSS (Figure 4B-E). No significant differences were observed in the levels of either DAPK or pDAPK^{S308} levels between control and DSS-treated mice. Although, the increase in total DAPK levels were moderate, the pDAPK^{S308} levels decreased drastically in AOM+DSS treated mice indicating that DAPK is activated during transformation (Figure 4F). In case of STAT3 activation, STAT3^{Y705} phosphorylation was increased by DSS-treatment whereas it decreased profoundly in AOM+DSS treated mice (Figure 4G) confirming the immunostainings in human tissues of UC and UCC. These data implicate the importance of both proteins in the course of inflammation-associated carcinogenesis.
To find out if this observation is a rather occasional phenomenon or if the two molecules control the expression of each other under inflammatory conditions, we developed an in vitro model to simulate the TNF-driven inflammatory process using the normal intestinal epithelial cell line HCEC. HCEC is an immortalized cell line developed by transfection of the SV40 large T antigen cDNA into freshly isolated human colon epithelial cells isolated from a non-tumor carrying donor.^{45,46} HCEC cells differ from cancer cells in that they are not tumorigenic as they did not develop tumors in SCID mice.³¹ We analysed the immunohistochemical expression of cytokeratin to verify their epithelial origin. As expected, all the cells were positive when stained with anti-pan-cytokeratin. HCEC cells were treated with TNF and the interaction between DAPK and STAT3 was characterised in detail.

### TNF induces an inflammatory pathway in HCEC cells

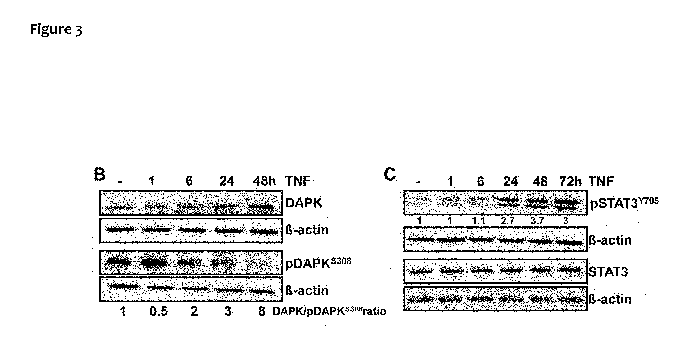

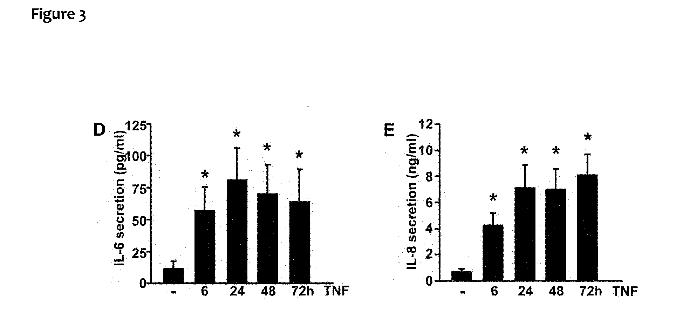

To find out whether an inflammatory stimulus can modulate the expression/activation of DAPK and STAT3, HCEC cells were treated with TNF for various time points. Interestingly, TNF caused the dephosphorylation of DAPK^{S308} (inactive form of DAPK) after 6 hours, reaching a maximum after 48 hours. In addition, DAPK expression was enhanced after 48 hours. A gradual increase in DAPK/pDAPKS³⁰⁸ ratio indicated that DAPK is activated upon TNF treatment (Figure 3B). in parallel, STAT3^{Y705} phosphorylation was significantly enhanced after a 24h-TNF treatment, whereas total STAT3 levels did not change (Figure 3C). As TNF is known to regulate the expression of other cytokines,¹⁹ IL-6 and IL-8 secretion was measured by ELISA. As expected, TNF significantly induced the secretion of both pro-inflammatory cytokines IL-6 (~7.2-fold) and IL-8 (~12-fold) from 6 to 72 hours (Figure 3D,E). This was accompanied by an increase in IL-6 and IL-8 mRNA levels at earlier time points but was rather marginally increased at 24 hours and later. To find out whether STAT3 is activated by the released IL-6, HCEC cells were treated with TNF in the presence or absence of anti-IL-6 monoclonal antibodies (mAB). TNF-induced STAT3^{Y705} phosphorylation was diminished by 30% after IL-6 neutralisation. In parallel, stimulation of cells with IL-6 induced STAT3^{Y705} phosphorylation after 30 minutes at the earliest time point and resumed after 48 hours. Other possible TNF-induced cellular functions like apoptosis or cell viability were not altered. Taken together, these results imply that an inflammatory pathway was activated in HCEC cells in response to TNF.

### TNF activation of DAPK and STAT3 in human colon cancer cells

To investigate if TNF-stimulation can alter the expression/activation of DAPK and STAT3 in cancer cells, HT-29 and DLD1 colorectal cancer cells were treated with TNF for various time points and assessed by Western blotting. In HT-29 cells, TNF-treatment caused a slight enhancement in the DAPK/pDAPK^{S308} ratio and pSTAT3^{Y705} protein level whereby pSTAT3^{Y705} was completely absent in the control cells. In DLD1 cells we also observed an increase in the DAPK/pDAPK^{S308} ratio (DAPK^{S308} phosphorylation decreased considerably) but the STAT3^{Y705} phosphorylation decreased (at 6 and 24 hours) and reached the control levels at 48 hours. When comparing pSTAT3^{Y705} protein level between both cell lines, the HT-29 cells expressed a general lower protein level than DLD1 cells before and after TNF-treatment. Obviously there seem to exist major differences in TNF-induced signaling between normal and tumor epithelial cells in vitro.

### DAPK negatively regulates the TNF-induced STAT3^{Y705} phosphorylation and IL-6 secretion

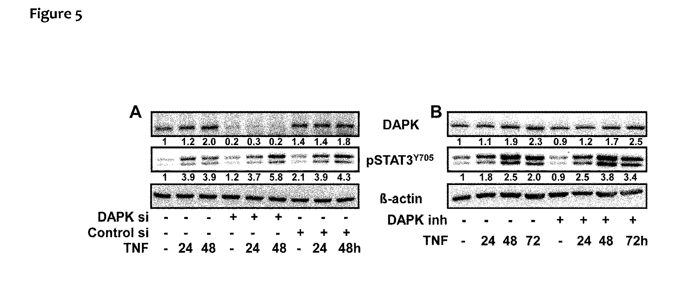

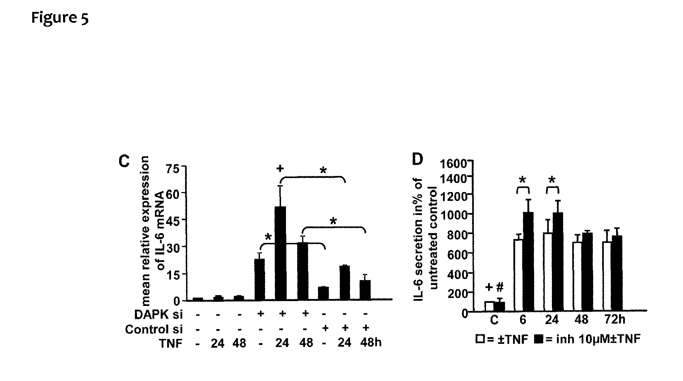

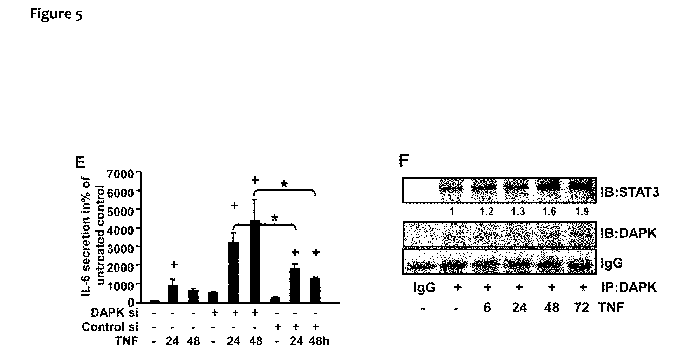

To investigate the role of DAPK in TNF-induced inflammation, a siRNA-mediated DAPK depletion experiment was performed. HCEC cells were transfected with DAPK siRNA or non-specific control siRNA and subsequently treated with TNF for 24 and 48 hours. DAPK expression was depleted by up to 85% in cells transfected with DAPK siRNA (Figure 5A). interestingly, TNF-induced STAT3^{Y705} phosphorylation was significantly enhanced after 48 hours (1-5-fold) following DAPK knockdown when compared to non-transfected and control siRNA transfected cells (Figure 5A). Then, mRNA expression of IL-6 was compared between TNF-treated and/or DAPK silenced HCEC cells. DAPK si knock down significantly induced IL-6 mRNA expression in untreated and TNF-treated cells at 24 and 48 hours in comparison to the corresponding control si RNA transfected and non-transfected cells (Figure 5C). In parallel, DAPK knock down potentiated TNF-induced secretion after 24 hours (1.7-fold) and 48 hours (3.4-fold) but not in untreated cells (Figure 5E). This suggests that DAPK per se has a clear effect on IL-6 mRNA expression but only TNF-treatment triggers DAPK to influence the secretion of this cytokine. These data further support the active part of DAPK in inflammation of the mucosal microenvironment. On the other hand DAPK knockdown had no effect on IL-8 secretion (data not shown).
In a separate experiment, the requirement of DAPK kinase activity to suppress the TNF-induced inflammatory process was assessed by treating the cells with TNF for various time points in the presence or absence of a specific DAPK kinase inhibitor.⁴⁷ Inhibition of DAPK catalytic activity significantly increased TNF-induced IL-6 secretion after 6 and 24 hours, whereas STAT3^{Y705} phosphorylation was enhanced later, i.e. after 24, 48, and 72 hours (Figure 5B,D). Notably, inhibition of DAPK catalytic activity potentiated TNF-induced IL-6 secretion, but not to the extent of DAPK depletion, thereby indicating that not only the kinase activity but also other functional domains of the kinase seem to be involved in regulating the TNF-induced inflammatory response.

### DAPK and STAT3 interaction is increased under TNF treatment

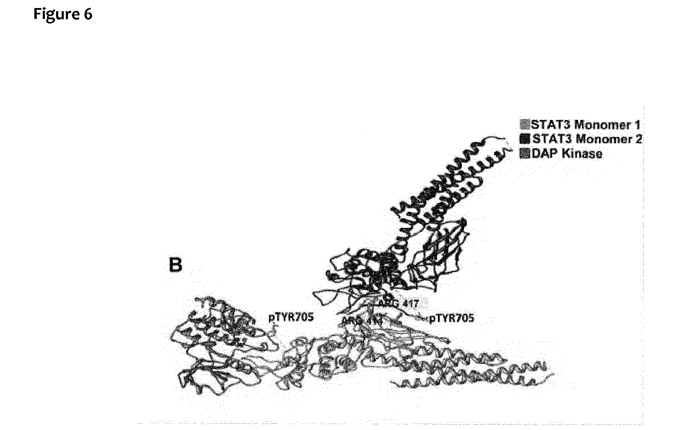

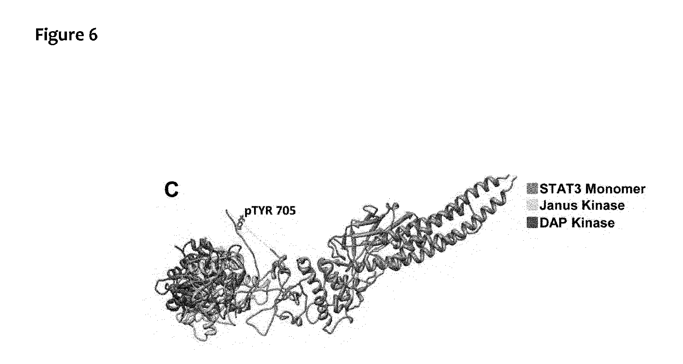

To further investigate if DAPK interacts with STAT3, DAPK was immunoprecipitated and blotted with anti-STAT3 antibodies. Indeed, we demonstrated a physical interaction of DAPK with STAT3, which was elevated under TNF treatment (Figure 5F). To understand the role of DAPK in this complex we used a 3D structural model to analyze DAPK-dependent conformational changes. Activated STAT3 formed a "butterfly-shaped" dimer (Figure 6A). The distance between the phosphorylated tyrosines (pY705) in the native STAT-STAT dimer was 37.1Å. This distance increased to 81.5Å in the presence of DAPK, and the original "butterfly"-structure was distorted by DAPK docking (Figure 6B). Furthermore, the analysis of hydrogen bond and hydrophobic interactions showed that DAPK seems to initiate new interactions (seven additional hydrogen bonds) between both molecules (Tables 3, 4). Interestingly, the 3D modelling showed that the DAPK binding region is nearly overlapping with that of the STAT3 upstream kinase JAK, thus suggesting a competition between both kinases for STAT3 binding (Figure 6C).

### TNF-activated STAT3 translocates into the nucleus and binds to the DAPK promoter

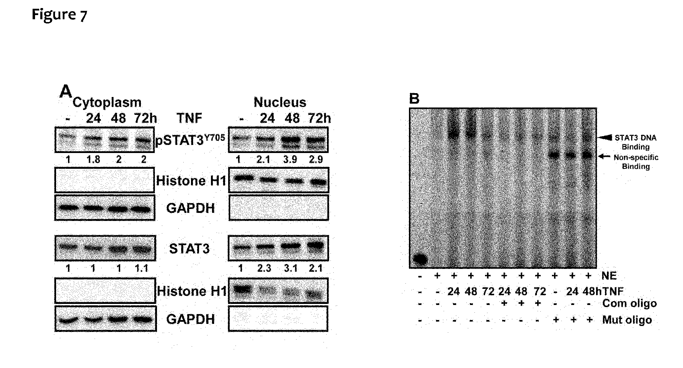

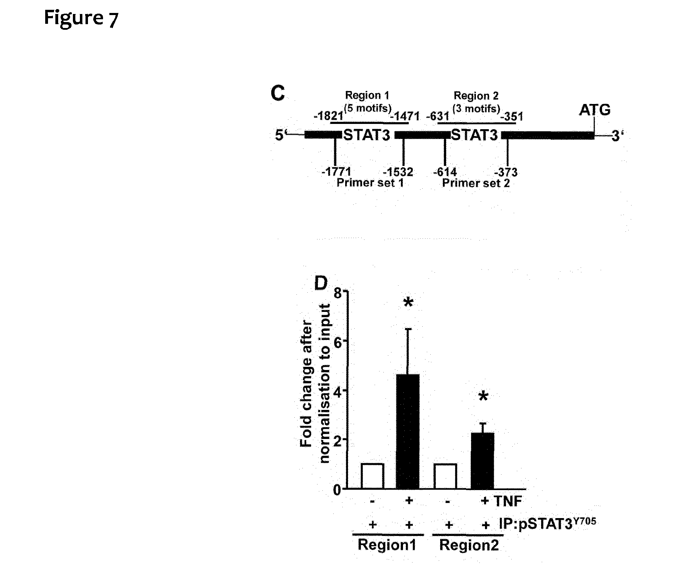

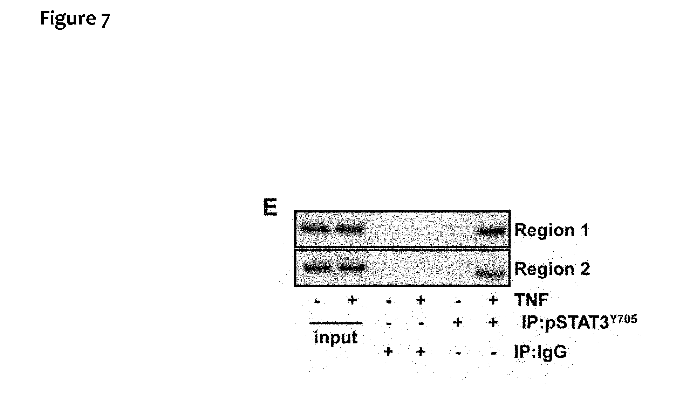

To examine whether TNF induces the nuclear translocation of STAT3, nuclear extracts were analyzed by Western blotting. As shown in figure 7A, increased levels of pSTAT3^{Y705} and STAT3 were observed in the nuclear fractions after 24, 48 or 72 hours of TNF treatment. To investigate whether TNF activated STAT3 transcriptional activity, EMSA assays were performed using labelled and unlabelled oligonucleotides containing a consensus or mutated STAT3 binding motif. No complexes were detected in the case of untreated cells. Protein-DNA complexes were prominent after the TNF treatment (after 24, 48 or 72 hours). This interaction was diminished when the extracts were incubated with an excess of cold unlabelled or mutant oligonucleotides (Figure 7B). A higher transcriptional transactivation is in agreement with the 3D structural model, in which the formation of STAT-STAT dimer showing an exposed nuclear localization signal (NLS) formed by the vital residues R414/R417 is essential for the shuttling of pSTAT3^{Y705} into the nucleus (Figure 6A). After docking of DAPK to the dimer, the NLS which favors the DNA binding is buried between the dimer's interface (Figure 6B). Thus, the shuttling of the pSTAT3^{Y705} to the nucleus and subsequently the activation of target genes might be blocked, explaining the remarkable increase in IL-6 mRNA expression after DAPK knock down.
Our sequence analysis of the DAPK promoter (Database of Transcriptional Start sites: DBTSS: NM_004938) revealed the presence of putative STAT3 binding motifs (TTN5AA or TTN6AA). The scheme of the DAPK promoter (Figure 7C) illustrates these putative STAT3 binding sites, five in Region1 (-1471 to -1821) and three in Region2 (-351 to -631). Next, ChIP experiments were performed to verify whether TNF induced the direct binding of pSTAT3^{Y705} to the DAPK promoter in *vivo.* Two different primer pairs, specific for each region, were designed. The analysis of precipitated DNA using qPCR and/or end-point PCR demonstrated that TNF augmented STAT3 binding to the DAPK promoter in both regions when compared to untreated cells. Immunoprecipitated DNA with negative control IgG could not be amplified (Figure 7D,E). These data suggest that STAT3 might regulate DAPK mRNA expression in response to TNF stimulation in normal IEC and verified DAPK as a new transcriptional target of STAT3.

### DAPK and IL-6 expression are regulated by STAT3

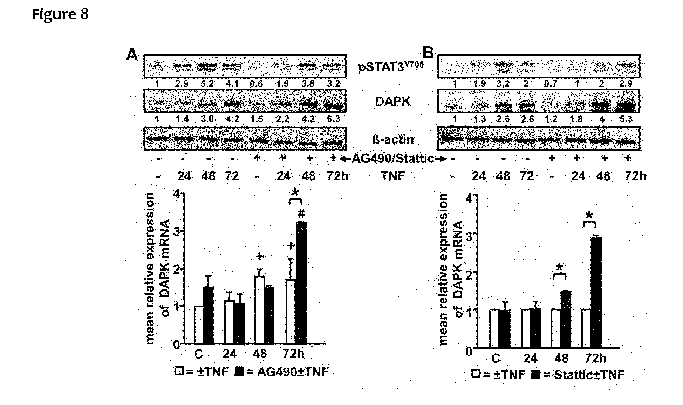

Previous studies have shown that STAT3 could either promote or suppress the expression of its target genes.^{40,48} To further evaluate STAT3 transcriptional regulation of DAPK expression, HCEC cells were stimulated with TNF in the presence or absence of AG490 (Janus kinase inhibitor) or Stattic (inhibits STAT3 phosphorylation and dimerisation). pSTAT3^{Y705} levels decreased significantly (by 35%; Figure 8A, upper panel) when cells were treated with AG490 prior to TNF treatment. Whereas TNF induced the expression of DAPK mRNA after 48 or 72 hours only by 1.5-fold, the STAT3 inactivation by AG490 pre-treatment increased the DAPK mRNA expression after 72 hours by 3.3-fold, thus suggesting a transcriptional repression of DAPK by STAT3 (Figure 8A, lowerpanel). Similarly, TNF-induced DAPK protein expression was elevated upon STAT3 inactivation (Figure 8A, upper panel).
TNF-induced STAT3^{Y705} phosphorylation was also down-regulated by Stattic pre-treatment (up to 45%). Similar to AG490, DAPK protein (Figure 8B, upper panel) as well as DAPK mRNA was enhanced after 48 hours (1-5-fold) and 72 hours (2.8-fold) in presence of Stattic (Figure 8B, lower panel). These results reveal that STAT3 activation restricts the TNF-increased DAPK expression and again suggests that STAT3 is a novel negative regulator of DAPK expression.
In parallel, we studied the effect of STAT3 inactivation on the expression of its already known target gene IL-6. Our data show that treatment with AG490 significantly down-regulated TNF-induced IL-6 mRNA expression as well as secretion by 50%, thereby indicating that TNF-induced IL-6 expression is positively regulated by STAT3.

### Schematic overview of TNF-induced signaling/functions in HCEC

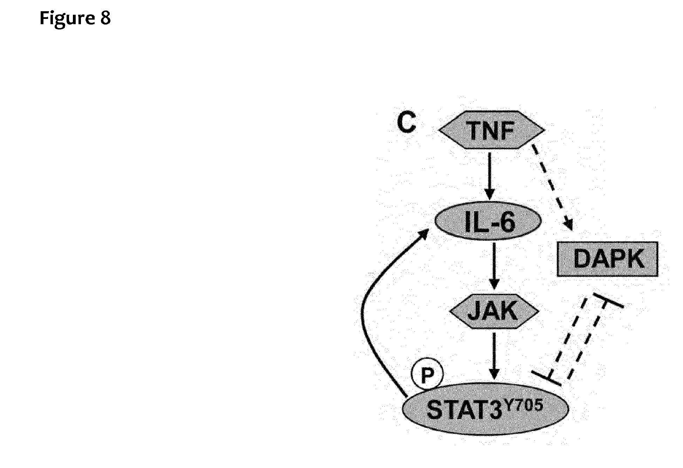

Based on our findings we propose the following working model (Figure 8C). TNF induces DAPK expression/activation which attenuates TNF-induced STAT3 activity either directly by physical interaction or indirectly by suppressing IL-6→STAT3 pathway. Vice versa, STAT3 represses DAPK expression at the transcriptional level. Activated STAT3 enhances IL-6 secretion, thereby forming a positive feed back loop. Finally, DAPK and STAT3 negatively regulate each other to promote their own expression/activation and most probably to balance the TNF-induced inflammatory signaling.

### DISCUSSION

Cellular response to the pro-inflammatory cytokine TNF varies depending on the cellular setting.⁴⁹ Our results demonstrate that DAPK expression and DAPK catalytic activity was increased in HCEC cells after TNF treatment. In parallel, TNF stimulation induced IL-6→STAT3-dependent inflammatory pathway. This is in agreement with earlier reports demonstrating the induction of an inflammatory cascade by TNF in different cell types.^{4,17,19,50-52} For the first time we show that both proteins, DAPK and STAT3 negatively regulate each other.
DAPK knockdown potentiated STAT3^{Y705} phosphorylation, IL-6 mRNA expression, and IL-6 secretion. Interestingly, DAPK knockdown enhanced IL-6 mRNA expression irrespective of TNF treatment, whereas the increased IL-6 secretion seems to be a clear TNF-dependent effect. These findings are in line with the recently identified suppressive function of DAPK in TCR- and LPS-triggered NFκB activation.^{13,14} Lungs and macrophages of DAPK^{-/-} mice secreted higher levels of IL-6 and CXCL1 in response to LPS.¹⁴ However, the exact mechanism by which DAPK regulates inflammatory signalling remains unclear. Here we show that, inhibiting DAPK kinase activity was less effective than DAPK knockdown in promoting TNF-induced IL-6/STAT3 activation. This suggests a structural involvement of the protein in suppressing inflammatory functions of TNF. Many DAPK interaction partners are phosphorylated by DAPK, and the catalytic activity of DAPK is required for functional consequences such as apoptosis or autophagy.⁵³ A recent paper by Chuang et *al*¹² suggests a structural role of DAPK in the assembly of the NLRP3 inflammasome. We found a physical interaction of DAPK with STAT3 by immunoprecipitation. Because Y705 is not the DAPK consensus motif (RxxS/T), we suggest a phosphorylation-independent mechanism by which DAPK can suppress TNF-induced inflammation. We can only speculate about the role of DAPK in STAT3 complex. It might i) mask the NLS of STAT3 to impede its nuclear translocation or ii) prevent the access of the upstream kinase JAK and the subsequent STAT3 dimerization. Structural modelling supports both theories. DAPK docking to the complex changes the conformation of the STAT3 dimer in such a way that the NLS R414/417 is masked. The residues R414/417 are located in the DNA binding domain of STAT3 and have been reported to be required for the nuclear translocation of STAT3. The mutants of R214/215 or R414/417 failed to enter the nucleus in response to EGF or IL-6. Furthermore, Mutations on R414/417 have been shown to destroy the DNA binding activity of STAT3.⁵⁴ The Y705 residues forming a cross-link in the dimer are separated from each other when DAPK is associated with the complex. In addition the binding regions for DAPK and JAK are completely overlapping, thereby suggesting a binding competition between both kinases. Therefore, our data indicate that DAPK might play an essential role in equilibrating TN F-induced IL-6/STAT3 functions.
We show that TNF-activated STAT3 translocated to the nucleus, where its DNA binding activity was enhanced. For the first time using the ChIP assay we identified DAPK as a transcriptional target of STAT3. STAT3 inhibition using AG490 or Stattic elevated TNF-induced DAPK expression, thus demonstrating that STAT3 activation transcriptionally represses DAPK. To date, the transcriptional regulation of DAPK expression is only poorly understood. A recent report shows that DAPK mRNA level is negatively regulated via the noncanonical FIt₃ITD/NFκB pathway.⁵⁵ Another study reported that IFNγ-induced DAPK expression was dependent on C/EBP-β.⁵⁶ Treatment of melanoma cells with 4-hydroxytamoxifen/oncostatin M-induced STAT3 activation and up-regulated DAPK mRNA transcription.⁵⁷ We have previously shown that promoter methylation leads to transcriptional silencing of DAPK in UC carcinogenesis and colorectal cancer.^{11,58} Indeed, in our study approximately 25% of UCC samples showed only a low or moderate immunohistochemical DAPK protein expression in the epithelium, thus suggesting an epigenetic regulation in these cases. Further studies are required to understand the association between methylation, inflammation and IL-6/STAT3 signalling.

There is only one report showing a positive feed back loop between IL-6 and STAT3 in autophagic cancer cells in which STAT3 directly binds to the IL-6 promoter.⁵⁹ We observed that blocking the IL-6/STAT3 pathway by IL-6 neutralization or by addition of the JAK inhibitor AG490 led to a decreased TNF-induced STAT3^{Y705} phosphorylation and IL-6 mRNA expression/secretion, thus indicating a positive feedback loop after TNF treatment. We suggest that IL-6 transduces the activation signal of STAT3 and in turn IL-6-activated STAT3 can contribute to IL-6 production in the inflammatory milieu of the epithelium. These data are consistent with previous reports describing that IL-6 and STAT3 co-operate with each other to enhance their activity.⁶⁰⁻⁶² Our recent studies reported the involvement of IL-6/STAT3 in the disease perpetuation of UC.²³ In contrast, deficient gp130/IL-6/STAT3 signalling in IEC increased their sensitivity to DSS-induced colitis showing that IL-6/STAT₃ pathway is also important for regulating epithelial turnover and mucosal healing to maintain gastrointestinal homeostasis.^{27,28,40,63} Finally, the mechanism whereby IL6/STAT3 increases colitis severity still remains unclear.⁶⁴
As enhanced levels of pro-inflammatory cytokines might cause instability in the balance of cell turnover leading to the development of aberrant crypt architecture,²⁹ the expression of the inflammation-associated proteins DAPK and pSTAT₃^{Y705} was evaluated in UC tissues. IHC results demonstrate that epithelial DAPK and pSTAT3^{Y705} expression increased to the stage of active colitis and correlated with the grade of inflammation as observed in our earlier studies.^{11,65} Other reports show that epithelial STAT3 activation correlates with the severity of colitis.^{53,66} Thus, DAPK and pSTAT3^{Y705} follow the same expression pattern from the inactive to the active colitis stage. However, the exact steps that follow the colitis-DALM-carcinoma sequence in between have never been analyzed in detail for both proteins. In DALM samples we found a heterogeneity allowing all possible combinations (Figure 2A). In UCC specimens DAPK levels remained high but pSTAT3^{Y705}levels decreased in comparison to those in active UC samples. Interestingly, pSTAT3^{Y705} levels were also found to be less in AOM+DSS-carcinomas when compared to DSS-colitis tissues. Both findings are is in accordance to Wick et *al,*⁶⁷ who reported a decrease of pSTAT3^{Y705} expression in UCC samples (0.75) when compared to UC samples (0.89) in their scoring system, and to Li Y *et al*,²⁹ who also showed this decrease of approximately 10%. Nevertheless, the limited sample size in the available studies encourages conducting further studies using larger number of samples.
In summary, our findings provide a novel molecular insight into the TNF-induced signaling network. TNF induced a dual signaling with simultaneous activation of an anti-inflammatory DAPK pathway and a pro-inflammatory STAT3 pathway. We suggest that normal cells may have developed mechanisms for reciprocal negative regulation of pro- and anti-inflammatory proteins to balance the inflammatory milieu.

### References

1. Baud V, Karin M: Signal transduction by tumor necrosis factor and its relatives. Trends Cell Biol 2001,11:372-7
2. MacEwan DJ: TNF receptor subtype signalling: differences and cellular consequences. Cell Signal 2002,14:477-92
3. Karin M, Gallagher E: TNFR signaling: ubiquitin-conjugated TRAFfic signals control stop-and-go for MAPK signaling complexes. Immunol Rev 2009, 228:225-40.
4. Bhattacharyya S, Dudeja PK, Tobacman JK: Tumor necrosis factor alpha-induced inflammation is increased but apoptosis is inhibited by common food additive carrageenan. J Biol Chem 2010, 285:39511-22.
5. Chen G, Goeddel DV. TNF-R1 signaling: a beautiful pathway. Science 2002, 296:1634-5..
6. Rutgeerts P, Van Assche G, Vermeire S: Review article: Infliximab therapy for inflammatory bowel disease--seven years on. Aliment Pharmacol Ther 2006, 23:451-63.
7. Ardizzone S, Bianchi Porro G: Inflammatory bowel disease: new insights into pathogenesis and treatment. J Intern Med. 2002, 252:475-96.
8. Fiocchi C: Inflammatory bowel disease: etiology and pathogenesis. Gastroenterology 1998,115:182-205.
9. Sartor RB: Mechanisms of disease: pathogenesis of Crohn's disease and ulcerative colitis. Nat Clin Pract Gastroenterol Hepatol 2006, 3:390-407.
10. Munkholm P: Review article: the incidence and prevalence of colorectal cancer in inflammatory bowel disease. Aliment Pharmacol Ther 2003, 2:1-5.
11. Kuester D, Guenther T, Biesold S, Hartmann A, Bataille F, Ruemmele P, Peters B, Meyer, Schubert D, Bohr UR, Malfertheiner P, Lippert H, Silver AR, Roessner A, Schneider-Stock R: Aberrant methylation of DAPK in long-standing ulcerative colitis and ulcerative colitis-associated carcinoma. Pathol Res Pract. 2010, 206:616-24
12. Chuang YT, Lin YC, Lin KH, Chou TF, Kuo WC, Yang KT, Wu PR, Chen RH, Kimchi A, Lai MZ: Tumor suppressor death-associated protein kinase is required for full IL-1β production. Blood. 2011,117:960-70.
13. Chuang YT, Fang LW, Lin-Feng MH, Chen RH, Lai MZ: The tumor suppressor death-associated protein kinase targets to TCR-stimulated NF-kappa B activation. J Immunol. 2008, 180:3238-49
14. Nakav S, Cohen S, Feigelson SW, Bialik S, Shoseyov D, Kimchi A, Alon R: Tumor suppressor death-associated protein kinase attenuates inflammatory responses in the lung. Am J Respir Cell Mol Biol 2012, 46:313-22
15. Ghosh S, May MJ, Kopp EB: NF-kappa B and Rel proteins: evolutionarily conserved mediators of immune responses. Annu Rev Immunol 1998, 16:225-60
16. Wajant H, Pfizenmaier K, Scheurich P: Tumor necrosis factor signaling. Cell Death Differ 2003,10:45-65
17. Zhong Z, Wen Z, Darnell JE Jr: Stat3: a STAT family member activated by tyrosine phosphorylation in response to epidermal growth factor and interleukin-6. Science 1994, 264:95-8
18. Becker C, Fantini MC, Schramm C, Lehr HA, Wirtz S, Nikolaev A, Burg J, Strand S, Kiesslich R, Huber S, Ito H, Nishimoto N, Yoshizaki K, Kishimoto T, Galle PR, Blessing M, Rose-John S, Neurath MF: TGF-beta suppresses tumor progression in colon cancer by inhibition of IL-6 trans-signaling. Immunity 2004, 21:491-501
19. Hodge DR, Hurt EM, Farrar WL: The role of IL-6 and STAT3 in inflammation and cancer. Eur J Cancer 2005, 41:2502-12
20. Meydan N, Grunberger T, Dadi H, Shahar M, Arpaia E, Lapidot Z, Leeder JS, Freedman M, Cohen A, Gazit A, Levitzki A, Roifman CM: Inhibition of acute lymphoblastic leukaemia by a Jak-2 inhibitor. Nature 1996, 379:645-8
21. Akira S: Roles of STAT3 defined by tissue-specific gene targeting. Oncogene 2000, 19:2607-11
22. Aggarwal BB, Kunnumakkara AB, Harikumar KB, Gupta SR, Tharakan ST, Koca C, Dey S, Sung B: Signal transducer and activator of transcription-3, inflammation, and cancer: how intimate is the relationship?. Ann N Y Acad Sci 2009,1171:59-76
23. Atreya R, Mudter J, Finotto S, Müllberg J, Jostock T, Wirtz S, Schütz M, Bartsch B, Holtmann M, Becker C, Strand D, Czaja J, Schlaak JF, Lehr HA, Autschbach F, Schürmann G, Nishimoto N, Yoshizaki K, Ito H, Kishimoto T, Galle PR, Rose-John S, Neurath MF: Blockade of interleukin 6 trans signaling suppresses T-cell resistance against apoptosis in chronic intestinal inflammation: evidence in crohn disease and experimental colitis in vivo. Nat Med 2000, 6:583-8
24. Becker C, Fantini MC, Wirtz S, Nikolaev A, Lehr HA, Galle PR, Rose-John S, Neurath MF: IL-6 signaling promotes tumor growth in colorectal cancer. Cell Cycle 2005, 4:217-20
25. Karin M, Greten FR: NF-kappaB: linking inflammation and immunity to cancer development and progression. Nat Rev Immunol 2005, 5:749-59
26. Corvinus FM, Orth C, Moriggl R, Tsareva SA, Wagner S, Pfitzner EB, Baus D, Kaufmann R, Huber LA, Zatloukal K, Beug H, Ohlschlager P, Schutz A, Halbhuber KJ, Friedrich K: Persistent STAT3 activation in colon cancer is associated with enhanced cell proliferation and tumor growth. Neoplasia 2005, 7:545-55
27. Bollrath J, Phesse TJ, von Burstin VA, Putoczki T, Bennecke M, Bateman T, Nebelsiek T, Lundgren-May T, Canli O, Schwitalla S, Matthews V, Schmid RM, Kirchner T, Arkan MC, Ernst M, Greten FR: gp130-mediated Stat3 activation in enterocytes regulates cell survival and cell-cycle progression during colitis-associated tumorigenesis. Cancer Cell 2009,15:91-102
28. Grivennikov S, Karin E, Terzic J, Mucida D, Yu GY, Vallabhapurapu S, Scheller J, Rose-John S, Cheroutre H, Eckmann L, Karin M: IL-6 and Stat3 are required for survival of intestinal epithelial cells and development of colitis-associated cancer. Cancer Cell 2009,15:103-13
29. Li Y, de Haar C, Chen M, Deuring J, Gerrits MM, Smits R, Xia B, Kuipers EJ, van der Woude CJ: Disease-related expression of the IL6/STAT3/SOCS3 signalling pathway in ulcerative colitis and ulcerative colitis-related carcinogenesis. Gut 2010, 59:227-35
30. Danese S: Nonimmune cells in inflammatory bowel disease: from victim to villain. Trends Immunol 2008, 29:555-64
31. Herbst U, Fuchs JI, Teubner W, Steinberg P: Malignant transformation of human colon epithelial cells by benzo[c]phenanthrene dihydrodiolepoxides as well as 2-hydroxyamino-1-methyl-6-phenylimidazo[4,5-b]pyridine. Toxicol Appl Pharmacol 2006, 212:136-45
32. Schroeder KW, Tremaine WJ, Ilstrup DM: Coated oral 5-aminosalcylic acid therapy for mildly to moderately active ulcerative colitis. N Eng J Med 1987, 317:1625-1629.
33. Poehlmann A, Habold C, Walluscheck D, Reissig K, Bajbouj K, Ullrich O, Hartig R, Gali-Muhtasib H, Diestel A, Roessner A, Schneider-Stock R: Cutting edge: Chk1 directs senescence and mitotic catastrophe in recovery from G2 checkpoint arrest. J Cell Mol Med 2011, 15:1528-41
34. Kozakov D, Hall DR, Beglov D, Brenke R, Comeau SR, Shen Y, Li K, Zheng J, Vakili P, Paschalidis ICh, Vajda S: Achieving reliability and high accuracy in automated protein docking: ClusPro, PIPER, SDU, and stability analysis in CAPRI rounds 13-19. Proteins 2010, 78:3124-30
35. Petsko GA, Ringe D: Bonds that stabilize folded proteins in Protein Structure and Function. Published by New Science Press 2004, pp.10-11
36. Rau TT, Rogler A, Frischauf M, Jung A, Konturek PC, Dimmler A, Faller G, Sehnert B, El-Rifai W, Hartmann A, Voll RE, Schneider-Stock R: Methylation-dependent activation of CDX1 through NF-κB: a link from inflammation to intestinal metaplasia in the human stomach. Am J Pathol 2012,181:487-98.
37. Gali-Muhtasib H, Kuester D, Mawrin C, Bajbouj K, Diestel A, Ocker M, Habold C, Foltzer-Jourdainne C, Schoenfeld P, Peters B, Diab-Assaf M, Pommrich U, Itani W, Lippert H, Roessner A, Schneider-Stock R: Thymoquinone triggers inactivation of the stress response pathway sensor CHEK1 and contributes to apoptosis in colorectal cancer cells. Cancer Res 2008, 68:5609-18
38. Takeda K, Kaisho T, Yoshida N, Takeda J, Kishimoto T, Akira S: Stat3 activation is responsible for IL-6-dependent T cell proliferation through preventing apoptosis: generation and characterization of T cell-specific Stat3-deficient mice. J Immunol 1998, 161:4652-60.
39. Madison BB, Dunbar L, Qiao XT, Braunstein K, Braunstein E, Gumucio DL: Cis elements of the villin gene control expression in restricted domains of the vertical (crypt) and horizontal (duodenum, cecum) axes of the intestine. J Biol Chem 2002, 277:33275-83
40. Pickert G, Neufert C, Leppkes M, Zheng Y, Wittkopf N, Warntjen M, Lehr HA, Hirth S, Weigmann B, Wirtz S, Ouyang W, Neurath MF, Becker C: STAT3 links IL-22 signaling in intestinal epithelial cells to mucosal wound healing. J Exp Med 2009, 206:1465-72
41. Neurath MF, Wittkopf N, Wlodarski A, Waldner M, Neufert C, Wirtz S, Günther C, Becker C: Assessment of tumor development and wound healing using endoscopic techniques in mice. Gastroenterology 2010,139:1837-1843
42. Becker C, Fantini MC, Neurath MF: High resolution colonoscopy in live mice. Nat Protoc. 2006,1:2900-4
43. Neufert C, Becker C, Neurath MF: An inducible mouse model of colon carcinogenesis for the analysis of sporadic and inflammation-driven tumor progression. Nat Protoc 2007, 2:1998-2004.
44. Bajbouj K, Poehlmann A, Kuester D, Drewes T, Haase K, Hartig R, Teller A, Kliche S, Walluscheck D, Ivanovska J, Chakilam S, Ulitzsch A, Bommhardt U, Leverkus M, Roessner A, Schneider-Stock R: Identification of phosphorylated p38 as a novel DAPK-interacting partner during TNFalpha-induced apoptosis in colorectal tumor cells. Am J Pathol 2009, 175:557-70
45. Blum S, Pfeiffer A, Tromvoukis Y: Immortalized adult human colon epithelial cell line. US Patent 2001, 6,194,203 B1
46. Duthie SJ, Narayanan S, Blum S, Pirie L, Brand GM: Folate deficiency in vitro induces uracil misincorporation and DNA hypomethylation and inhibits DNA excision repair in immortalized normal human colon epithelial cells. Nutr Cancer 2000, 37:245-51
47. Okamoto M, Takayama K, Shimizu T, Muroya A, Furuya T: Structure-activity relationship of novel DAPK inhibitors identified by structure-based virtual screening. Bioorg Med Chem 2010, 18:2728-34
48. Snyder M, Huang XY, Zhang JJ: Identification of novel direct Stat3 target genes for control of growth and differentiation. J Biol Chem 2008, 283:3791-8
49. Kyriakis JM: Life-or-death decisions. Nature. 2001, 414:265-6
50. Kunisch E, Gandesiri M, Fuhrmann R, Roth A, Winter R, Kinne RW: Predominant activation of MAP kinases and pro-destructive/pro-inflammatory features by TNF alpha in early-passage synovial fibroblasts via TNF receptor-1: failure of p38 inhibition to suppress matrix metalloproteinase-1 in rheumatoid arthritis. Ann Rheum Dis 2007, 66:1043-51
51. Scharl M, McCole DF, Weber A, Vavricka SR, Frei P, Kellermeier S, Pesch T, Fried M, Rogler G: Protein tyrosine phosphatase N2 regulates TNFα-induced signalling and cytokine secretion in human intestinal epithelial cells. Gut 2011, 60:189-97
52. Kamitani S, Togi S, Ikeda O, Nakasuji M, Sakauchi A, Sekine Y, Muromoto R, Oritani K, Matsuda T: Krüppel-associated box-associated protein 1 negatively regulates TNF-α-induced NF-κB transcriptional activity by influencing the interactions among STAT3, p300, and NF-κB/p65. J Immunol 2011,187:2476-83
53. Lin Y, Hupp TR, Stevens C: Death-associated protein kinase (DAPK) and signal transduction: additional roles beyond cell death. FEBS J 2010, 277:48-57
54. Ma J, Zhang T, Novotny-Diermayr V, Tan AL, Cao X. A novel sequence in the coiled-coil domain of Stat3 essential for its nuclear translocation. J Biol Chem 2003, 278:29252-60
55. Shanmugam R, Gade P, Wilson-Weekes A, Sayar H, Suvannasankha A, Goswami C, Li L, Gupta S, Cardoso AA, AI Baghdadi T, Sargent KJ, Cripe LD, Kalvakolanu DV, Boswell HS: A noncanonical FIt3ITD/NF-κB signaling pathway represses DAPK1 in acute myeloid leukemia. Clin Cancer Res 2012, 18:360-9
56. Gade P, Roy SK, Li H, Nallar SC, Kalvakolanu DV: Critical role for transcription factor C/EBP-beta in regulating the expression of death-associated protein kinase 1. Mol Cell Biol 2008, 28:2528-48
57. Schick N, Oakeley EJ, Hynes NE, Badache A: TEL/ETV6 is a signal transducer and activator of transcription 3 (Stat3)-induced repressor of Stat3 activity. J Biol Chem 2004, 279:38787-96
58. Mittag F, Kuester D, Vieth M, Peters B, Stolte B, RoessnerA, Schneider-Stock R: DAPK promotor methylation is an early event in colorectal carcinogenesis. Cancer Lett 2006, 240:69-75
59. Yoon S, Woo SU, Kang JH, Kim K, Kwon MH, Park S, Shin HJ, Gwak HS, Chwae YJ: STAT3 transcriptional factor activated by reactive oxygen species induces IL6 in starvation-induced autophagy of cancer cells. Autophagy 2010, 6:1125-38
60. Sumimoto H, Imabayashi F, Iwata T, Kawakami Y: The BRAF-MAPK signaling pathway is essential for cancer-immune evasion in human melanoma cells. J Exp Med 2006, 203:1651-6
61. Fritzenwanger M, Meusel K, Foerster M, Kuethe F, Krack A, Figulla HR: Cardiotrophin-1 induces interleukin-6 synthesis in human umbilical vein endothelial cells. Cytokine 2006, 36:101-6
62. Fritzenwanger M, Meusel K, Foerster M, Kuethe F, Krack A, Figulla HR: Cardiotrophin-1 induces interleukin-6 synthesis in human monocytes. Cytokine 2007, 38:137-44
63. Tebbutt NC, Giraud AS, Inglese M, Jenkins B, Waring P, Clay FJ, Malki S, Alderman BM, Grail D, Hollande F, Heath JK, Ernst M: Reciprocal regulation of gastrointestinal homeostasis by SHIP2 and STAT-mediated trefoil gene activation in gp130 mutant mice. Nat Med 2002, 8:1089-97
64. Suzuki A, Hanada T, Mitsuyama K, Yoshida T, Kamizono S, Hoshino T, Kubo M, Yamashita A, Okabe M, Takeda K, Akira S, Matsumoto S, Toyonaga A, Sata M, Yoshimura A: CIS3/SOCS3/SSI3 plays a negative regulatory role in STAT3 activation and intestinal inflammation. J Exp Med 2001, 193:471-81
65. Mudter J, Weigmann B, Bartsch B, Kiesslich R, Strand D, Galle PR, Lehr HA, Schmidt J, Neurath MF: Activation pattern of signal transducers and activators of transcription (STAT) factors in Inflammatory bowel diseases. Am J Gastroenterol 2005, 100:64-72.
66. Carey R, Jurickova I, Ballard E, Bonkowski E, Han X, Xu H, Denson LA: Activation of an IL-6:STAT3-dependent transcriptome in pediatric-onset inflammatory bowel disease. inflamm Bowel Dis 2008,14:446-57
67. Wick EC, Leblanc RE, Ortega G, Robinson C, Platz E, Pardoll DM, lacobuzio-Donahue C, Sears CL: Shift from pStat6 to pStat3 predominance is associated with inflammatory bowel disease-associated dysplasia. Inflamm Bowel Dis 2011, .doi: 10.1002/ibd.21908.
68. Kuester D, Guenther T, Biesold S, Hartmann A, Bataille F, Ruemmele P, Peters B, Meyer, Schubert D, Bohr UR, Malfertheiner P, Lippert H, Silver AR, Roessner A, Schneider-Stock R: Aberrant methylation of DAPK in long-standing ulcerative colitis and ulcerative colitis-associated carcinoma. Pathol Res Pract. 2010, 206:616-24
69. Chuang YT, Lin YC, Lin KH, Chou TF, Kuo WC, Yang KT, Wu PR, Chen RH, Kimchi A, Lai MZ: Tumor suppressor death-associated protein kinase is required for full IL-1β production. Blood. 2011,117:960-70.
70. Chuang YT, Fang LW, Lin-Feng MH, Chen RH, Lai MZ: The tumor suppressor death-associated protein kinase targets to TCR-stimulated NF-kappa B activation. J Immunol. 2008, 180:3238-49
71. Nakav S, Cohen S, Feigelson SW, Bialik S, Shoseyov D, Kimchi A, Alon R: Tumor suppressor death-associated protein kinase attenuates inflammatory responses in the lung. Am J Respir Cell Mol Biol 2012, 46:313-22
72. Munkholm P: Review article: the incidence and prevalence of colorectal cancer in inflammatory bowel disease. Aliment Pharmacol Ther 2003, 2:1-5.

- Cohen O, Feinstein E, Kimchi A. DAP-kinase is a Ca2+/calmodulin-dependent, cytoskeletal-associated protein kinase, with cell death-inducing functions that depend on its catalytic activity. EMBO J 1997,16:998-1008.
- Eisenberg-Lerner A, Kimchi A. PKD at the crossroads of necrosis and autophagy. Autophagy 2012, 8:433-4.
- Gandesiri M, Chakilam S, Ivanovska J, Benderska N, Ocker M, Di Fazio P, Feoktistova M, Gali-Muhtasib H, Rave-Fränk M, Prante O, Christiansen H, Leverkus M, Hartmann A, Schneider-Stock, R. DAPK plays an important role in panobinostat-induced autophagy and commits cells to apoptosis under autophagy deficient conditions. Apoptosis 2012,17:1300-1315
- Inbal B, Shani G, Cohen O, Kissil JL, Kimchi A: Death-associated protein kinase-related protein 1, a novel serine/threonine kinase involved in apoptosis. Mol Cell Biol 2000, 20:1044-54.
- Kuester D, Guenther T, Biesold S, Hartmann A, Bataille F, Ruemmele P, Peters B, Meyer, Schubert D, Bohr UR, Malfertheiner P, Lippert H, Silver AR, Roessner A, Schneider-Stock R: Aberrant methylation of DAPK in long-standing ulcerative colitis and ulcerative colitis-associated carcinoma. Pathol Res Pract. 2010, 206:616-24.
- Mittag F, Kuester D, Vieth M, Peters B, Stolte B, Roessner A, Schneider-Stock R: DAPK promotor methylation is an early event in colorectal carcinogenesis. Cancer Lett 2006, 240:69-75.
- Neufert C, Becker C, and Neurath M F. An inducible mouse model of colon carcinogenesis for the analysis of sporadic and inflammation-driven tumor progression. Nat Protoc 2007, 2:1998-2004.
- Gandesiri, M., Chakilam, S., Ivanovska, J., Benderska, N., Ocker, M., Di Fazio, P., Feoktistova, M., Gali-Muhtasib, H., Rave-Fränk, M., Prante, O., Christiansen, H., Leverkus, M., Hartmann, A., Schneider-Stock, R. DAPK plays an important role in panobinostat-induced autophagy and commits cells to apoptosis under autophagy deficient conditions. Apoptosis. 2012. 17:1300-15.
- Neurath M F, Wittkopf N, Wlodarski A, Waldner M, Neufert C, Wirtz S, Günther C, Becker C. Assessment of tumor development and wound healing using endoscopic techniques in mice. Gastroenterology. 2010, 39:1837-1843.

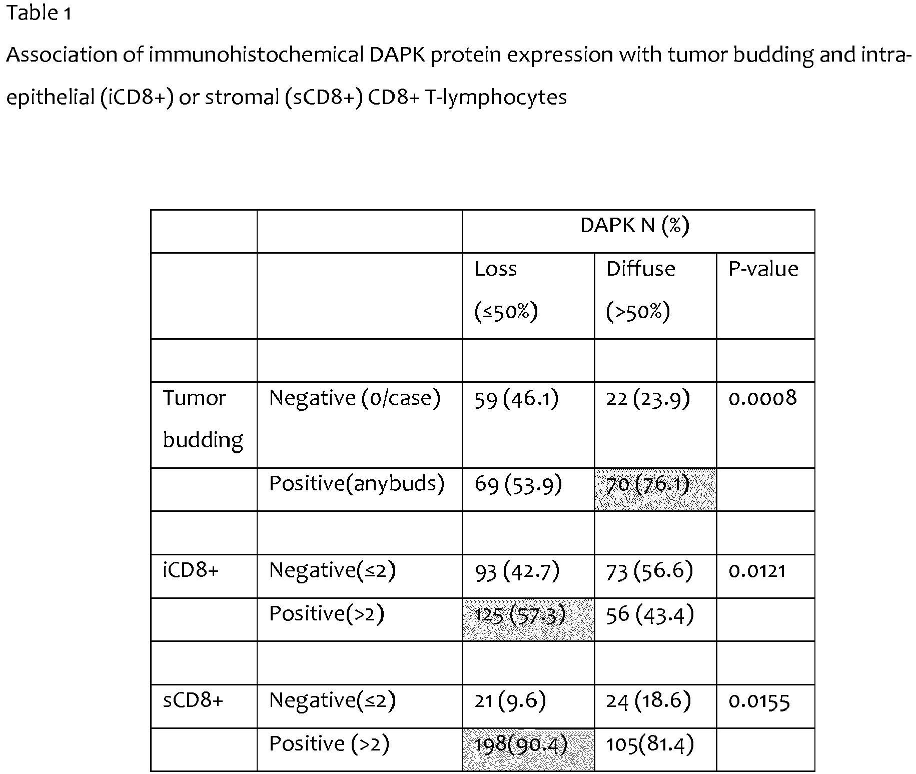

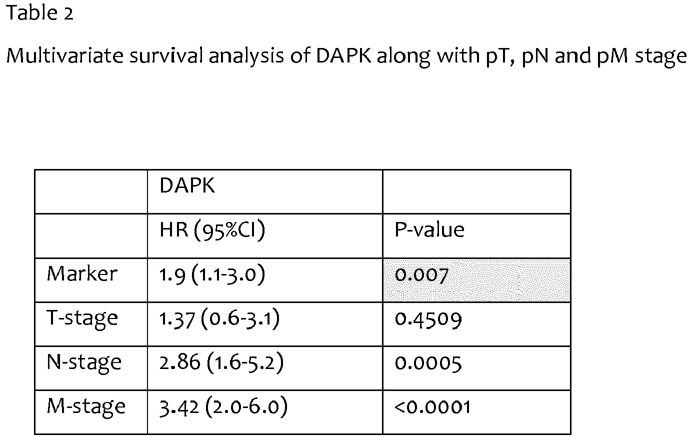

**Table 3**

| Analysis of hydrogen bonding interaction patterns in the STAT3-STAT3 dimer and in the STAT3-DAPK-STAT3 complex. | | | |
|---|---|---|---|
| **STAT3-STAT3** | | **STAT3-DAPK-STAT3** | |
| **STAT3 Monomer 1** | **STAT3 monomer 2** | **STAT3 Monomer 1** | **STAT3 monomer 2** |
| Glu 638 | Asn 664 | **Arg 414** | **Pro 639, Glu 638, Gln 644** |
| Asn 647 | Lys 709 | **Arg 417** | **Tyr 640, Thr 714, Cys 712, Val 713** |
| Ser 649 | Thr 708 | Glu 415 | Tyr 640 |
| Ser 649 | Leu 706 | Ser 465 | Pro 715 |
| Glu 652 | Thr 708 | Gln 416 | Asn 647 |
| Arg 688 | Leu 706 | Asn 385 | Gln 644, Asn 647, |
| Leu 706 | Glu 652, Phe 710, Cys 712 | Gln 469 | Phe 716 |
| | | Arg 423 | Leu 666 |
| | | Asp 374 | Thr 708 |
| | | Asn 420 | Glu 652, Lys 709, Met 655 |
| | | Arg 379 | Lys 709 |

**Table 4**

| Analysis of hydrophobic interactions between STAT3-STAT3 dimer and the STAT3-DAPK-STAT3 complex. | | | |
|---|---|---|---|
| **STAT3-STAT3** | | **STAT3-DAPK-STAT3** | |
| **STAT3 Monomer 1** | **STAT3 monomer 2** | **STAT3 Monomer 1** | **STAT3 monomer 2** |
| Phe 710 | Met 648 | Leu 666 | Ala 428 |
| Ala 703 | Ala 578, Leu 577 | Leu 706 | Ala 376 |
| Pro 704 | Leu 577 | Phe 710 | Leu 378 |
| | | Pro 715 | Phe 384, Val 432 |

## Claims

1. A pharmaceutical composition comprising a therapeutically effective amount of an inhibitor of DAPK or DAPK or an activator of DAPK, and a pharmaceutically acceptable carrier for use in a method of treating or preventing or delaying cancer.

2. The pharmaceutical composition of claim 1, wherein said inhibitor of DAPK is selected from an inhibitor of DAPKalpha or beta, preferably DAPKalpha, or an activator of DAPKalpha or beta, preferably of DAPKbeta.

3. The pharmaceutical composition of claim 1 or 2, wherein said inhibitor of DAPK is selected from an antibody, complimentary nucleotide sequences or antisense sequences inhibiting DAPK expression or a compound represented by the general formula (1), 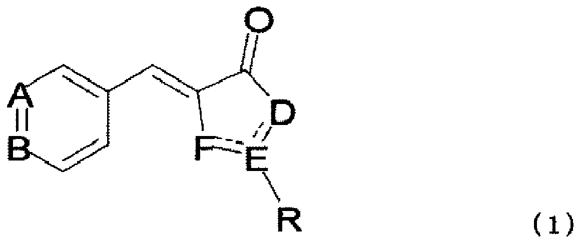
in which one of A and B represents a nitrogen atom and the other a carbon atom, D, E and F represent the combination O-C=N or N=C-S,
and R represents a substituted or unsubstituted, linear, branched or cyclic, saturated or unsaturated lower alkyl group or aralkyl group,
a substituted or unsubstituted aryl group, or a linear, branched or cyclic, substituted or unsubstituted, saturated or unsaturated aminoalkylgroup,
preferably wherein R represents a linear, branched or cyclic, optionally substituted lower alkyl group, an optionally substituted saturated or unsaturated aralkyl group, an optionally substituted aryl group, or a linear, branched or cyclic substituted or unsubstituted aminoalkylgroup,
particularly preferably wherein R represents a linear, branched or cyclic lower alkylgroup, a saturated or unsaturated lower aralkyl group, a linear, branched or cyclic aminoalkyl group or a group of formula (2), 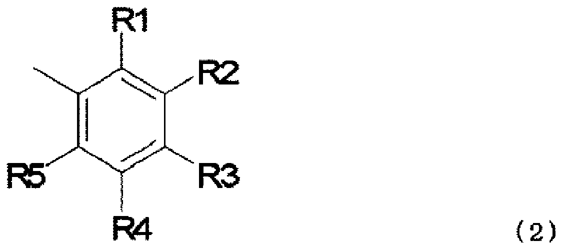
in which R1 to R5 independently represent a hydrogen atom, a halogen atom, a nitro group, a lower alkyl group or a lower alkoxy group;
or a salt, solvate, hydrate, active metabolite or prodrug thereof.

4. The pharmaceutical composition of any one of the previous claims, wherein said cancer is selected from colon cancer, colorectal cancer, gastro-intestinal tract cancer or ulcerative colitis-associated cancer and wherein said inflammation is ulceritis or ulcerative colitis.

5. An in vitro method for determining the risk of developing cancer in a subject comprising determining the expression level of DAPK, specifically DAPKalpha, in a sample potentially associated with cancer of said subject, wherein if the expression level of DAPK, specifically DAPKalpha, is increased with respect to a reference value, said subject has an increased risk of developing cancer, or wherein if the expression level of DAPK, specifically DAPKalpha, is decreased with respect to a reference value, said subject has a decreased risk of developing cancer, or
an in vitro method for determining the risk of developing cancer in a subject comprising determining the expression level of DAPK, specifically DAPKbeta, in a sample potentially associated with cancer of said subject, wherein if the expression level of DAPK, specifically DAPkbeta, is increased with respect to a reference value, said subject has an decreased risk of developing cancer, or wherein if the expression level of DAPkbeta is decreased with respect to a reference value, said subject has an increased risk of developing cancer.

6. An in vitro method for determining the overall survival time of a subject suffering from cancer comprising determining the expression level of DAPK, specifically DAPKalpha, in a tumor sample of said subject, wherein if the expression level of DAPK, specifically DAPKalpha, is increased with respect to a reference value, said subject has a shorter survival time, or wherein if the expression level of DAPK, specifically DAPKalpha, is decreased with respect to a reference value, said subject has an increased and disease-free survival time, or
an in vitro method for determining the overall survival time of a subject suffering from cancer comprising determining the expression level of DAPK, specifically DAPKbeta in a tumor sample of said subject, wherein if the expression level of DAPK, specifically DAPKbeta, is increased with respect to a reference value, said subject has a higher survival time, or wherein if the expression level of DAPK, specifically DAPKbeta, is decreased with respect to a reference value, said subject has a decreased survival time.

7. An in vitro method for designing a personalized therapy for a subject suffering from cancer comprising determining the expression level of DAPK, specifically DAPKalpha, in a tumor sample of said subject wherein if the expression level of DAPK, specifically DAPKalpha, is increased with respect to a reference value, then a therapy directed to prevent and/or treat said cancer is selected, or
an in vitro method for designing a personalized therapy for a subject suffering from cancer comprising determining the expression level of DAPK, specifically DAPKbeta, in a tumor sample of said subject, wherein if the expression level of DAPK, specifically DAPKbeta, is decreased with respect to a reference value, then a therapy directed to prevent and/or treat said cancer is selected.

8. The in vitro method of claim 5 or 6 or 7, wherein said cancer is selected from colon cancer, colorectal cancer, gastro-intestinal tract cancer or ulcerative colitis-associated cancer and wherein said inflammation is selected from ulceritis, ulcerative colitis.

9. The in vitro method according to any one of claims 5 to 8, wherein the determination of the expression level comprises the determination of the mRNA level of said gene, the determination of the cDNA level or determining the protein level encoded by said gene.

10. The in vitro method according to any one of claims 5 to 9, wherein the determination of the expression level is carried out by quantitative PCR, in situ hybridization or by a cDNA array.

11. The in vitro method according to any one of claims 5 to 10, wherein the determination of the protein level is carried out by western blot or immunohistochemistry.

12. The in vitro method according to claim 7, wherein said therapy is a chemotherapy, radiotherapy or gene therapy and/or wherein said personalized therapy comprises a predictive or therapeutic response prediction, optionally measurement as a biomarker the follow-up response to therapy.

13. A kit comprising means for detection the presence and the amount of DAPK in a sample of a subject supposed to suffer from cancer in order to perform a method according to anyone of claims 5 - 12.

14. A pharmaceutical composition comprising a therapeutically effective amount of DAPK and/or an activator or inhibitor of DAPK and a pharmaceutically acceptable carrier for use in a method of inducing or suppressing inflammation.

15. The pharmaceutical composition of claim 14, wherein said activator or inhibitor of DAPK is selected from an activator or inhibitor of DAPKalpha or beta, preferably DAPKbeta.
